# EUROPEAN PATENT APPLICATION

(11) **EP 2 977 463 A1**
(43) Date of publication of application: **27.01.2016**
(21) Application number: 14382286.4
(22) Date of filing: 25.07.2014
(51) Int. Cl.: C12Q 1/68

(54) **Methods and agents related to lung diseases**

(71) Applicant: Centro de Investigación Biomédica en Red (CIBER), 28029 Madrid (ES); Fundación Para la Investigación Biomédica del Hospital Universitario de Getafe, 28905 Getafe (ES)
(72) Inventor: Lorente Balanza, José Ángel, 28035 Madrid (ES); Cardinal Fernández, Pablo, 28901 Getafe (ES); Ferruelo Alonso, Antonio, 28523 Rivas Vaciamadrid (ES)
(74) Representative: ABG Patentes, S.L.

(57) **Abstract**

The invention relates to the field of lung diseases therapeutics, particularly to diagnostic and prognostic methods of lung pathologies including diffuse alveolar damage (DAD) and acute respiratory distress syndrome (ARDS), as well as to agent useful in the treatment and/or prevention of DAD in a subject.

SEQUENCE LISTING

<110>  CENTRO DE INVESTIGACIÓN BIOMÉDICA EN RED (CIBER)
       FUNDACIÓN PARA LA INVESTIGACIÓN BIOMÉDICA DEL HOSPITAL
       UNIVERSITARIO DE GETAFE

<120>  METHODS AND AGENTS RELATED TO LUNG DISEASES

<130>  P10602EP00

<160>  42

<170>  PatentIn version 3.5

<210>  1
<211>  22
<212>  RNA
<213>  Homo sapiens

<400>  1
agggcuuagc ugcuugugag ca                                              22

<210>  2
<211>  22
<212>  RNA
<213>  Homo sapiens

<400>  2
uccuguacug agcugccccg ag                                              22

<210>  3
<211>  20
<212>  RNA
<213>  Bos taurus

<400>  3
guaucccacu ucugacacca                                                 20

<210>  4
<211>  20
<212>  RNA
<213>  Mus musculus

<400>  4
ugugugugug ugugugugug                                                 20

<210>  5
<211>  22
<212>  RNA
<213>  Homo sapiens

<400>  5
acggguuagg cucuugggag cu                                              22

<210>  6
<211>  22
<212>  RNA
<213>  Homo sapiens

<400>  6
uucauucggc uguccagaug ua                                              22


<210>  7
<211>  22
<212>  RNA
<213>  Homo sapiens

<400>  7
accguggcuu ucgauuguua cu                                              22


<210>  8
<211>  22
<212>  RNA
<213>  Mus musculus

<400>  8
aaccguggcu uucgauuguu ac                                              22


<210>  9
<211>  22
<212>  RNA
<213>  Homo sapiens

<400>  9
uuuggucccc uucaaccagc ug                                              22


<210>  10
<211>  22
<212>  RNA
<213>  Homo sapiens

<400>  10
uaacacuguc ugguaacgau gu                                              22


<210>  11
<211>  22
<212>  RNA
<213>  Homo sapiens

<400>  11
auaagacgag caaaaagcuu gu                                              22


<210>  12
<211>  18
<212>  RNA
<213>  Rattus norvegicus

<400>  12
auaagacgag caaaaagc                                                   18


<210>  13
<211>  21
<212>  RNA
<213>  Homo sapiens

<400>  13
caacaccagu cgaugggcug u                                                21

<210>  14
<211>  22
<212>  RNA
<213>  Mus musculus

<400>  14
caacagcagu cgaugggcug uc                                               22

<210>  15
<211>  22
<212>  RNA
<213>  Homo sapiens

<400>  15
uggcaguguc uuagcugguu gu                                               22

<210>  16
<211>  22
<212>  RNA
<213>  Mus musculus

<400>  16
uuuguucguu cggcucgcgu ga                                               22

<210>  17
<211>  22
<212>  RNA
<213>  Mus musculus

<400>  17
gcucgacuca ugguuugaac ca                                               22

<210>  18
<211>  23
<212>  RNA
<213>  Rattus norvegicus

<400>  18
agcucgacuc augguuugaa cca                                              23

<210>  19
<211>  22
<212>  RNA
<213>  Homo sapiens

<400>  19
caaccuggag gacuccaugc ug                                               22

<210>  20
<211>  21
<212>  RNA
<213>  Homo sapiens

<400>  20
uacugcagac aguggcaauc a                                                21

<210>  21
<211>  22
<212>  RNA
<213>  Mus musculus

<400>  21
uacuccagaa uguggcaauc au                                               22

<210>  22
<211>  23
<212>  RNA
<213>  Rattus norvegicus

<400>  22
uacuccagaa uauggcaauc aug                                              23

<210>  23
<211>  21
<212>  RNA
<213>  Rattus norvegicus

<400>  23
agaggcuuau agcucuaagc c                                                21

<210>  24
<211>  72
<212>  RNA
<213>  Homo sapiens

<400>  24
ugucggguag cuuaucagac ugauguugac uguugaaucu cauggcaaca ccagucgaug      60

ggcugucuga ca                                                          72

<210>  25
<211>  78
<212>  RNA
<213>  Homo sapiens

<400>  25
cugaggagca gggcuuagcu gcuugugagc aggguccaca ccaagucgug uucacagugg      60

cuaaguuccg cccccag                                                     78

<210>  26
<211>  110
<212>  RNA
<213>  Homo sapiens

<400>  26
ggccagcugu gaguguuucu uuggcagugu cuuagcuggu uguugugagc aauaguaagg      60

aagcaaucag caaguauacu gcccuagaag ugcugcacgu uguggggccc                110

<210>  27
<211>  88
<212>  RNA
<213>  Homo sapiens

<400>  27
ugcgcuccuc ucagucccug agacccuaac uugugauguu uaccguuuaa auccacgggu      60

uaggcucuug ggagcugcga gucgugcu                                          88


<210>  28
<211>  101
<212>  RNA
<213>  Homo sapiens

<400>  28
ccgcccccgc gucuccaggg caaccguggc uuucgauugu uacuguggga acuggaggua      60

acagucuaca gccauggucg ccccgcagca cgcccacgcg c                          101


<210>  29
<211>  87
<212>  RNA
<213>  Rattus norvegicus

<400>  29
caaugcuuug cuaaagcugg uaaaauggaa ccaaaucgcc ucuucaaugg auuugguccc      60

cuucaaccag cuguagcuau gcauuga                                           87


<210>  30
<211>  90
<212>  RNA
<213>  Homo sapiens

<400>  30
ccgggccccu gugagcaucu uaccggacag ugcuggauuu cccagcuuga cucuaacacu      60

gucugguaac gauguucaaa ggugacccgc                                        90


<210>  31
<211>  71
<212>  RNA
<213>  Homo sapiens

<400>  31
ugacgggcga gcuuuuggcc cggguuauac cugaugcuca cguauaagac gagcaaaaag      60

cuuguugguc a                                                            71


<210>  32
<211>  64
<212>  RNA
<213>  Homo sapiens

<400>  32

ccccgcgacg agccccucgc acaaaccgga ccugagcguu uuguucguuc ggcucgcgug 60

aggc 64

<210> 33
<211> 94
<212> RNA
<213> Mus musculus

<400> 33
ucgacucugg guuugaacca aagcucgacu caugguuuga accauuacuu aauucguggu 60

uugaaccauc acucgacucc ugguucgaac cauc 94

<210> 34
<211> 121
<212> RNA
<213> Mus musculus

<400> 34
agagaucaug ucagugaacu gccacauuuu ugauauauau uauguaugua ugugugugug 60

ugugugugug uguauauaua uacacacaca cauacacacu acauguaugc aacauauaua 120

c 121

<210> 35
<211> 68
<212> RNA
<213> Homo sapiens

<400> 35
gcauccugua cugagcugcc ccgaggcccu ucaugcugcc cagcucgggg cagcucagua 60

caggauac 68

<210> 36
<211> 128
<212> RNA
<213> Homo sapiens

<400> 36
uggaggccuu gcugguuugg aaaguucauu guucgacacc auggaucucc agguggguca 60

aguuuagaga ugcaccaacc uggaggacuc caugcuguug agcuguucac aagcagcgga 120

cacuucca 128

<210> 37
<211> 94
<212> RNA
<213> Homo sapiens

<400> 37
caugcugugu gugguacccu acugcagaca guggcaauca uguauaauua aaaaugauug 60

guacgucugu ggguagagua cugcaugaca caug 94

<210>  38
<211>  76
<212>  RNA
<213>  Mus musculus

<400>  38
uucuaggucc agaggcuuau agcucuaagc cuuggaugaa agaggcuuau ggcuucaagc      60

uuucggagcu ggagac                                                      76


<210>  39
<211>  112
<212>  RNA
<213>  Homo sapiens

<400>  39
agacgaggag uuaagaguuc auucggcugu ccagauguau ccaaguaccc uguguuauuu      60

ggcaauaaau acaucugggc aacugacuga acuuuucacu uuucaugacu ca             112


<210>  40
<211>  98
<212>  RNA
<213>  Mus musculus

<400>  40
gagggguuaa gaguucauuc ggcuguccag auguacccaa guacccuguu auuuggcaau      60

acauacaucu gggcaacuga uugaacuuuu cgcuucuc                              98


<210>  41
<211>  139
<212>  RNA
<213>  Rattus norvegicus

<400>  41
ggcaaacuga agaaguguug gagagaugag ggguuaagag uucauucggc uguccagaug      60

uauccaagua cccuguuauu uggcaauaaa uacaucuggg caacugauug aacuuuucgc     120

uucucuugac ucaacugga                                                  139


<210>  42
<211>  77
<212>  RNA
<213>  Bos taurus

<400>  42
guuggggguc gguggugagc aagcagagac uacauagguu gccuuguauc ccacuucuga      60

caccauguau ugucaac                                                     77

## Description

### FIELD OF THE INVENTION

The invention relates to the field of lung diseases therapeutics, particularly to diagnostic and prognostic methods of lung pathologies including diffuse alveolar damage (DAD) and acute respiratory distress syndrome (ARDS), to prognostic methods suitable for subjects underdoing mechanical ventilation, as well as to agents useful in the treatment and/or prevention of DAD in a subject.

### BACKGROUND OF THE INVENTION

The acute respiratory distress syndrome (ARDS) is a serious and common disease in intensive care units (ICU), associated with a mortality of 40%. Diffuse alveolar damage (DAD) is considered the gold standard for histopathological confirmation of the syndrome. The American/European consensus for the classification of idiopathic interstitial pneumonias (AJRCCM 2002, 165:277-304) clearly defined that DAD and acute interstitial pneumonia (AIP) are histologically indistinguishable entities, suggesting the use of the term AIP for those cases where the etiology is not identified.

The main histopathological findings of AIP or DAD are: hyaline membrane formation (diffuse or patchy), diffuse distribution of the lesions within the alveolus, thickening of alveolar septal by organized fibrosis and generally diffuse airspace organization (patchy or diffuse). These findings should be presented in absence of granulomas, necrosis or abscesses, infectious agents (absence of viral inclusions and negative staining for microorganisms, absence of neutrophils and prominent eosinophils and negative cultures).

Some studies (Esteban A et al. 2004 Ann Intern Med 141:440-445; de Hemptinne Q et al. Chest 2009 135: 944-999; Pinheiro BV et al. 2007 J Bras Pneumol 33: 423-428; Thille AW et al. Am J Respir Crit Care Med 2013 187(7): 761-767) have analyzed the correlation between the clinical diagnosis of ARDS and the presence of DAD. All these studies found that the proportion of patients with a clinical diagnosis of ARDS and that showed DAD was less than 50%, which means that those patients presenting the clinical syndrome ARDS usually do not show the characteristic histological changes.

Additionally, all clinical trials aimed at the treatment of ARDS (except strategies intended to reduce ventilatory transpulmonary pressure and perhaps positioning prone patients) have to date yielded negative for its effect on mortality.

Thus, it is difficult to diagnose ARDS with DAD in clinical conditions (ie, identify patients who present DAD histologically in conditions wherein performing a lung biopsy is associated with elevated risks).

The search of biomarkers has been oriented to date, however, with the purpose of identifying the clinical syndrome (ARDS). Innovative orientation of the research is necessary in this context in order to identify biomarkers that indicate the presence of DAD, discriminating between patients with ARDS, those patients who do not have the characteristic histological changes of those with DAD.

Extravascular lung water content has been suggested as a post-mortem diagnostic tool for DAD (Tagami T et al. 2013 Crit Care Med 41(9): 2144-2150). Similarly, cytological features of bronchoalveolar lavage have been suggested in the diagnosis of DAD (Beskow CO et al. 2000 Acta Cytol 44(4): 640-646). The number of haemosiderin-laden macrophages in bronchoalveolar lavage fluid of patients of DAD has been analysed as a candidate marker for the diagnosis of DAD, although it was concluded that the diagnosis of DAD and DAH (diffuse alveolar hemorrhage) could be mistaken based on said marker (Maldonado F et al. 2009 Eur Respir J 33: 1361-1366).

Thus, there is still a need in the art to identify reliable diagnostic markers for diagnosis of DAD in a subject. It is therefore necessary to find a DAD biomarker for early and specific diagnosis of the entity. It is expected that the use of this biomarker (i) increases the specificity of the clinical diagnosis; (ii) allows a better selection of patients for testing innovative treatments; (iii) helps monitoring the response to treatment; (iv) helps developing risk stratification of unfavorable outcomes.

### SUMMARY OF THE INVENTION

The authors of the present invention have identified a group of miRNAs differentially expressed in subjects undergoing DAD in comparison to control subjects. From these miRNA, miR-27a-5p and miR-486-5p showed high sensitivity and specificity in DAD diagnosis. The miRNAs identified by the inventors also have a diagnostic value in the confirmation of ARDS diagnosis in a subject, as well as a prognostic value in a subject undergoing mechanical ventilation. Further, the inventors proposed agents inducing or inhibiting miRNA expression useful in the prevention and/or treatment of DAD in a subject.

Therefore, the invention relates to an *in vitro* method for the diagnosis of diffuse alveolar damage (DAD) in a subject that comprises
(i) determining the level of expression of at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof in a sample from the subject,
(ii) comparing the level of expression of said at least one miRNA to a reference value, and
(iii) correlating a variation in the expression level of said at least one miRNA respect to its reference value with the diagnosis of DAD.

In another aspect, the invention relates to an *in vitro* method for confirming diagnosis of acute respiratory distress syndrome (ARDS) in a subject suspected to suffer from ARDS that comprises:
(i) determining the level of expression of at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof in a sample from the subject,
(ii) comparing the level of expression of said at least one miRNA to a reference value, and
(iii) correlating a variation in the expression level of said at least one miRNA respect to its reference value with the confirmation of the diagnosis of ARDS in said subject.

In another aspect, the invention relates to an *in vitro* method for determining the prognosis of a subject undergoing mechanical ventilation that comprises
(i) determining the level of expression of at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof in a sample from the subject,
(ii) comparing the level of expression of said at least one miRNA to a reference value, and
(iii) correlating a variation in the expression level of said at least one miRNA respect to its reference value with the prognosis of said subject undergoing chemical ventilation.

In another aspect, the invention relates to an agent for use in the prevention and/or treatment of DAD in a subject selected from:
- an agent inhibiting the expression of a miRNA selected from the group consisting of miR-27a-5p, miR-486-5p, miR-2478, miR-125b-1-3p, miR-132-5p, miR-133a-3p, miR-208a-3p, miR-21-3p and miR-490-3p, and
- an agent inducing the expression of a miRNA selected from the group consisting of miR-1298, miR-200a-3p, miR-34a-5p, miR-375-3p, miR-509-5p, miR-879-5p, miR-466i-5p and miR 434-5p.

### BRIEF DESCRIPTION OF THE FIGURES

**Figure 1** shows a patient classification based on miRNA-27a-5p expression levels.
**Figure 2** shows the distribution of miR-27a-5p expression levels in rats ventilated with low tidal volume (control), rats ventilated with high tidal volume without DAD, and rats ventilated with high tidal volume with DAD.
**Figure 3** shows (A) the ROC curves corresponding to the discriminatory accuracy between animals with DAD and without DAD based on miR-27a-5p and miR-486-5p expression levels in rat serum samples. (B) miR-27a-5p and miR-486-5p expression levels determined in human lung samples.
**Figure 4** shows serum miR-27a-5p expression levels determined in subjects suffering from ARDS.
**Figure 5** shows the mortality ROC curve when miRNA-27a-5p expression level and/or SAPS II were analyzed.
**Figure 6** shows the mortality ROC curve when miRNA-27a-5p expression level, SAPS II, lactate level and procalcitonine level were analyzed.

### DETAILED DESCRIPTION OF THE INVENTION

### 1. Definitions

The term "administration", as used herein, relates to the delivery of a pharmaceutical drug to a subject.

The term "acute respiratory distress syndrome" (abbreviated ARDS), as used herein, relates to a severe, life-threatening medical condition characterized by presence of a risk factor (e.g. pneumonia, sepsis, pancreatitis, etc.), bilateral pulmonary infiltrates, and oxygen impairment not fully explained by cardiac failure. More particularly, the term ARDS as used herein relates to acute respiratory distress syndrome as convened in 2011 in the Berlin definition (ARDS Definition Task Force et al. 2012 JAMA 307(23): 2526-2533).

The term "agent inducing expression" or "inducer agent", as used in this invention, refers to any molecule that is capable of causing an increase in the transcription of a gene. Usually, the gene the transcription of which is induced in response to said inducer agent is under the operative control of a transcription regulatory region which, in turn, has binding sites for a transcription activator the activity of which increases in the presence of said inducer agent. Preferably, the inducer agent is a compound that is easy to administer and easily distributed in the body, and innocuous at the doses used to activate the system. Moreover, it must be capable of penetrating into the desired tissue or organ, and have a suitable half-life (usually of several hours).

The term "agent inhibiting expression", as used herein, relates to any molecule that is capable of causing a decrease in the transcription of a gene. Preferably, the inhibitor agent is a compound that is easy to administer and easily distributed in the body, and innocuous at the doses used to activate the system. Moreover, it must be capable of penetrating into the desired tissue or organ, and have a suitable half-life (usually of several hours).

The term "anti-miR", as used herein, also known as antagomir or blockmir, relates to a small synthetic RNA that is substantially complementary to the specific miRNA target with either mispairing at the cleavage site of Ago2 or some sort of base modification to inhibit Ago2 cleavage.

The term "decreased expression level", as used herein in relation to the expression level of a miRNA, relates to a situation where the level of expression of the miRNA is decreased at least 5%, at least 10%, at least 20%, at least 30%, at least 40%, at least 50%, at least 60%, at least 70%, at least 80%, at least 90% or at least 100% when compared to the corresponding reference value.

The term "diagnosis", as it is used herein, generally relates to the process by which a disease, nosological entity, syndrome, or any disease-health condition is identified. Particularly, the term "diagnosis of diffuse alveolar damage" relates to the capacity to identify or detect the presence of DAD; this detection, as it is understood by a person skilled in the art, does not claim to be correct in 100% of the analyzed samples. However, it requires that a statistically significant amount of the analyzed samples are classified correctly. The amount that is statistically significant can be established by a person skilled in the art by means of using different statistical tools; illustrative, nonlimiting examples of said statistical tools include determining confidence intervals, determining the p-value, the Student's t-test or Fisher's discriminant functions, etc. (see, for example, Dowdy and Wearden, Statistics for Research, John Wiley & Sons, New York 1983). The confidence intervals are preferably at least 90%, at least 95%, at least 97%, at least 98% or at least 99%. The p-value is preferably less than 0.1, less than 0.05, less than 0.01, less than 0.005 or less than 0.0001. The teachings of the present invention preferably allow correctly diagnosing DAD in at least 60%, in at least 70%, in at least 80%, or in at least 90% of the subjects of a determined group or population analyzed.

The term "diffuse alveolar damage" (abbreviated DAD), as used herein, relates to an histopatological pattern in lung disease. In a particular embodiment, DAD is associated to sepsis, infection, trauma, pancreatitis, bacterial pneumonia, viral pneumonia, multiple blood transfer, or high risk surgery post-operative.

The term "increased expression level", as used herein in relation to the expression level of a miRNA, relates to a situation where the level of expression of the miRNA is increased at least 5%, at least 10%, at least 20%, at least 30%, at least 40%, at least 50%, at least 60%, at least 70%, at least 80%, at least 90% or at least 100% when compared to the corresponding reference value.

The term "level of expression" relates to the measurement of the amount of a nucleic acid, e.g. RNA or mRNA, or of a protein. In the context of the invention, the level of expression relates to the measurement of the amount of a nucleic acid, particularly RNA, more particularly miRNA, even more particularly a miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof.

The term "mechanical ventilation", as used herein, relates to a method to mechanically assist or replace spontaneous breathing. It may involve a ventilator or the breathing may be assisted by a registered nurse, physician, respiratory therapist, paramedic, or other suitable person compressing a bag or set of bellows. Mechanical ventilation is considered to be invasive if it involves any instrument penetrating through the mouth (such as an endotracheal tube) or the skin (such as a tracheostomy tube).

The terms "miRNA" or "microRNA", used interchangeably herein, are endogenous RNAs, some of which are known to regulate the expression of protein-coding genes at the post-transcriptional level. As used herein, the term "miRNA" refers to any type of micro-interfering RNA, including but not limited to, endogenous microRNA and artificial microRNA. Typically, endogenous miRNAs are small RNAs encoded in the genome which are capable of modulating the productive utilization of mRNA. A mature miRNA is a single-stranded RNA molecule of about 21-23 nucleotides in length which is complementary to a target sequence, and hybridizes to the target RNA sequence to inhibit its translation. miRNAs themselves are encoded by genes that are transcribed from DNA but not translated into protein (non-coding RNA); instead they are processed from primary transcripts known as pri-miRNA to short stem-loop structures called pre-miRNA and finally to functional miRNA. Mature miRNA molecules are partially complementary to one or more messenger RNA (mRNA) molecules, and their main function is to down-regulate gene expression.

Pre-miRNAs are referred to as "mir", whereas mature miRNAs are referred to as "miR". This prefix is followed by a dash and a number, the latter often indicating order of naming. miRNAs with nearly identical sequences except for one or two nucleotides are annotated with an additional lower case letter. Pre-miRNAs that lead to 100% identical mature miRNAs but that are located at different places in the genome are indicated with an additional dash-number suffix. Species of origin is designated with a three-letter prefix. Nomenclature conventions used to describe miRNA sequences are described further in Ambros V et al. 2003 RNA 9:277-279.

The term "miR-21-3p", as used herein, refers to the human miRNA as defined in the miRBase database of the University of Manchester (http://www.mirbase.org/index.shtml) (release 21 as of June 2014) under accession number MIMAT0004494, as well as to homologs thereof including, without limitation, those defined in the above mentioned database that include mouse miR-21-3p (accession number MIMAT0004628, miR-21a-3p) and rat miR-21-3p (accession number MIMAT0004711). The nucleic acid sequences corresponding pre-miRNAs include, without limitation, human mir-21 (accession number MI0000077, UGUCGGGUAGCUUAUCAGACUGAUGUUGACUGUUGAAUCUCAUGGCAAC ACCAGUCGAUGGGCUGUCUGACA, SEQ ID NO:24).

The term "miR-27a-5p", as used herein, refers to the human miRNA as defined in the miRBase database of the University of Manchester (http://www.mirbase.org/index.shtml) (release 21 as of June 2014) under accession number MIMAT0004501, as well as to homologs thereof including, without limitation, those defined in the above mentioned database that include mouse miR-27a-5p (accession number MIMAT0004633) and rat miR-27a-5p (accession number MIMAT0004715). The nucleic acid sequences corresponding to pre-miRNAs include, without limitation, human mir-27a (accession number MI0000085, CUGAGGAGCAGGGCUUAGCUGCUUGUGAGCAGGGUCCACACCAAGUCGU GUUCACAGUGGCUAAGUUCCGCCCCCCAG, SEQ ID NO:25).

The term "miR-34a-5p", as used herein, refers to the human miRNA as defined in the miRBase database of the University of Manchester (http://www.mirbase.org/index.shtml) (release 21 as of June 2014) under accession number MIMAT0000255, as well as to homologs thereof including, without limitation, those defined in the above mentioned database that include mouse miR-34a-5p (accession number MIMAT0000542) and rat miR-34a-5p (accession number MIMAT0000815). The nucleic acid sequences corresponding to pre-miRNAs include, without limitation, human mir-34a (accession number MI0000268, GGCCAGCUGUGAGUGUUUCUUUGGCAGUGUCUUAGCUGGUUGUUGUGAG CAAUAGUAAGGAAGCAAUCAGCAAGUAUACUGCCCUAGAAGUGCUGCAC GUUGUGGGGCCC, SEQ ID NO:26).

The term "miR-125b-1-3p", as used herein, refers to the human miRNA as defined in the miRBase database of the University of Manchester (http://www.mirbase.org/index.shtml) (release 21 as of June 2014) under accession number MIMAT0004592, as well as to homologs thereof including, without limitation, those defined in the above mentioned database that include mouse miR-125b-1-3p (accession number MIMAT0004669) and rat miR-125b-1-3p (accession number MIMAT0004730). The nucleic acid sequences corresponding to pre-miRNAs include, without limitation, human mir-125b-1 (accession number MI0000446, UGCGCUCCUCUCAGUCCCUGAGACCCUAACUUGUGAUGUUUACCGUUUAA AUCCACGGGUUAGGCUCUUGGGAGCUGCGAGUCGUGCU, SEQ ID NO: 27).

The term "miR-132-5p", as used herein, refers to the human miRNA as defined in the miRBase database of the University of Manchester (http://www.mirbase.org/index.shtml) (release 21 as of June 2014) under accession number MIMAT0004594, as well as to homologs thereof including, without limitation, those defined in the above mentioned database that include mouse miR-132-5p (accession number MIMAT0016984) and rat miR-132-5p (accession number MIMAT0017123). The nucleic acid sequences corresponding to pre-miRNAs include, without limitation, human mir-132 (accession number MI0000449, CCGCCCCCGCGUCUCCAGGGCAACCGUGGCUUUCGAUUGUUACUGUGGGA ACUGGAGGUAACAGUCUACAGCCAUGGUCGCCCCGCAGCACGCCCACGCG C, SEQ ID NO: 28).

The term "miR-133a-3p", as used herein, refers to the human miRNA as defined in the miRBase database of the University of Manchester (http://www.mirbase.org/index.shtml) (release 21 as of June 2014) under accession number MIMAT0000427, as well as to homologs thereof including, without limitation, those defined in the above mentioned database that include mouse miR-133a-3p (accession number MIMAT0000145) and rat miR-133a-3p (accession number MIMAT0000839). The nucleic acid sequences corresponding to pre-miRNAs include, without limitation, rat mir-133a (accession number MI0000906, CAAUGCUUUGCUAAAGCUGGUAAAAUGGAACCAAAUCGCCUCUUCAAUG GAUUUGGUCCCCUUCAACCAGCUGUAGCUAUGCAUUGA, SEQ ID NO:29).

The term "miR-200a-3p", as used herein, refers to the human miRNA as defined in the miRBase database of the University of Manchester (http://www.mirbase.org/index.shtml) (release 21 as of June 2014) under accession number MIMAT0000682, as well as to homologs thereof including, without limitation, those defined in the above mentioned database that include mouse miR-200a-3p (accession number MIMAT0000519) and rat miR-200a-3p (accession number MIMAT0000874). The nucleic acid sequences corresponding to pre-miRNAs include, without limitation, human mir-200a (accession number MI0000737, CCGGGCCCCUGUGAGCAUCUUACCGGACAGUGCUGGAUUUCCCAGCUUGA CUCUAACACUGUCUGGUAACGAUGUUCAAAGGUGACCCGC, SEQ ID NO:30).

The term "miR-208a-3p", as used herein, refers to the human miRNA as defined in the miRBase database of the University of Manchester (http://www.mirbase.org/index.shtml) (release 21 as of June 2014) under accession number MIMAT0000241, as well as to homologs thereof including, without limitation, those defined in the above mentioned database that include mouse miR-208a-3p (accession number MIMAT0000520) and rat miR-208a-3p (accession number MIMAT0000880). The nucleic acid sequences corresponding to pre-miRNAs include, without limitation, human mir-208a (accession number MI0000251, UGACGGGCGAGCUUUUGGCCCGGGUUAUACCUGAUGCUCACGUAUAAGA CGAGCAAAAAGCUUGUUGGUCA, SEQ ID NO:31).

The term "miR-375-3p", as used herein, refers to miRNA as defined in the miRBase database of the University of Manchester (http://www.mirbase.org/index.shtml) (release 21 as of June 2014) under accession number MIMAT0005307, as well as to homologs thereof including, without limitation, those defined in the above mentioned database that include mouse miR-375-3p (accession number MIMAT0000739 and human miR-375-3p. The nucleic acid sequences corresponding to pre-miRNAs include, without limitation, human mir-375 (accession number MI0000783, CCCCGCGACGAGCCCCUCGCACAAACCGGACCUGAGCGUUUUGUUCGUUC GGCUCGCGUGAGGC, SEQ ID NO:32).

The term "miR-434-5p", as used herein, refers to miRNA as defined in the miRBase database of the University of Manchester (http://www.mirbase.org/index.shtml) (release 21 as of June 2014) under accession number MIMAT0017307, as well as to homologs thereof including, without limitation, those defined in the above mentioned database that include mouse miR-434-5p (accession number MIMAT0001421) and human miR-434-5p. The nucleic acid sequences corresponding to pre-miRNAs include, without limitation, mouse mir-434 (accession number MI0001526, UCGACUCUGGGUUUGAACCAAAGCUCGACUCAUGGUUUGAACCAUUACU UAAUUCGUGGUUUGAACCAUCACUCGACUCCUGGUUCGAACCAUC, SEQ ID NO:33).

The term "miR-466i-5p", as used herein, refers to miRNA as defined in the miRBase database of the University of Manchester (http://www.mirbase.org/index.shtml) (release 21 as of June 2014) under accession number MIMAT0017325, as well as to homologs thereof including, without limitation, human miR-466i-5p. The nucleic acid sequences corresponding to pre-miRNAs include, without limitation, mouse mir-466i (accession number MI0006282, AGAGAUCAUGUCAGUGAACUGCCACAUUUUUGAUAUAUAUUAUGUAUGU AUGUGUGUGUGUGUGUGUGUGUGUAUAUAUAUACACACACACAUACACA CUACAUGUAUGCAACAUAUAUAC, SEQ ID NO:34).

The term "miR-486-5p", as used herein, refers to the human miRNA as defined in the miRBase database of the University of Manchester (http://www.mirbase.org/index.shtml) (release 21 as of June 2014) under accession number MIMAT0002177, as well as to homologs thereof including, without limitation, those defined in the above mentioned database that include mouse miR-486-5p (accession number MIMAT0003130). The nucleic acid sequences corresponding to pre-miRNAs include, without limitation, human mir-486-1 (accession number MI0002470, GCAUCCUGUACUGAGCUGCCCCGAGGCCCUUCAUGCUGCCCAGCUCGGGG CAGCUCAGUACAGGAUAC, SEQ ID NO:35).

The term "miR-490-3p", as used herein, refers to the human miRNA as defined in the miRBase database of the University of Manchester (http://www.mirbase.org/index.shtml) (release 21 as of June 2014) under accession number MIMAT0002806, as well as to homologs thereof including, without limitation, those defined in the above mentioned database that include mouse miR-490-3p (accession number MIMAT0003780) and rat miR-490-3p (accession number MIMAT0012823). The nucleic acid sequences corresponding to pre-miRNAs include, without limitation, human mir-490 (accession number MI0003125, UGGAGGCCUUGCUGGUUUGGAAAGUUCAUUGUUCGACACCAUGGAUCUC CAGGUGGGUCAAGUUUAGAGAUGCACCAACCUGGAGGACUCCAUGCUGU UGAGCUGUUCACAAGCAGCGGACACUUCCA, SEQ ID NO:36).

The term "miR-509-5p", as used herein, refers to the human miRNA as defined in the miRBase database of the University of Manchester (http://www.mirbase.org/index.shtml) (release 21 as of June 2014) under accession number MIMAT0004779, as well as to homologs thereof including, without limitation, those defined in the above mentioned database that include mouse miR-509-5p (accession number MIMAT0004890) and rat miR-509-5p (accession number MIMAT0024849). The nucleic acid sequences corresponding to pre-miRNAs include, without limitation, human mir-509-1 (accession number MI0003196, CAUGCUGUGUGUGGUACCCUACUGCAGACAGUGGCAAUCAUGUAUAAUU AAAAAUGAUUGGUACGUCUGUGGGUAGAGUACUGCAUGACACAUG, SEQ ID NO:37).

The term "miR-879-5p", as used herein, refers to miRNA as defined in the miRBase database of the University of Manchester (http://www.mirbase.org/index.shtml) (release 21 as of June 2014) under accession number MIMAT0005287, as well as to homologs thereof including, without limitation, those defined in the above mentioned database that include mouse miR-879-5p (accession number MIMAT0004842) and human miR-879-5p. The nucleic acid sequences corresponding to pre-miRNAs include, without limitation, mouse mir-879 (accession number MI0005472, UUCUAGGUCCAGAGGCUUAUAGCUCUAAGCCUUGGAUGAAAGAGGCUUA UGGCUUCAAGCUUUCGGAGCUGGAGAC, SEQ ID NO:38).

The term "miR-1298", as used herein, refers to the human miRNA as defined in the miRBase database of the University of Manchester (http://www.mirbase.org/index.shtml) (release 21 as of June 2014) under accession number MIMAT0005800 (miR-1298-5p), as well as to homologs thereof including, without limitation, those defined in the above mentioned database that include mouse miR-1298 (accession number MIMAT0014809, miR-1298-5p) and rat miR-1298 (accession number MIMAT0025060). The nucleic acid sequences corresponding to pre-miRNAs include, without limitation, human mir-1298 under miRBase release 21 accession number MI0003938 (AGACGAGGAGUUAAGAGUUCAUUCGGCUGU CCAGAUGUAUCCAAGUACCCUGUGUUAUUUGGCAAUAAAUACAUCUGGG CAACUGACUGAACUUUUCACUUUUCAUGACUCA, SEQ ID NO:39), mouse mir-1298 under accession number MI0004300 (GAGGGGUUAAGAGUUCAUUCGGC UGUCCAGAUGUACCCAAGUACCCUGUUAUUUGGCAAUACAUACAUCUGG GCAACUGAUUGAACUUUUCGCUUCUC, SEQ ID NO:40) and rat mir-1298 under accession number MI0021845 (GGCAAACUGAAGAAGUGUUGGAGAGAUGA GGGGUUAAGAGUUCAUUCGGCUGUCCAGAUGUAUCCAAGUACCCUGUUA UUUGGCAAUAAAUACAUCUGGGCAACUGAUUGAACUUUUCGCUUCUCUU GACUCAACUGGA, SEQ ID NO:41).

The term "miR-2478", as used herein, refers to the miRNA as defined in the miRBase database of the University of Manchester (http://www.mirbase.org/index.shtml) (release 21 as of June 2014) under accession number MIMAT0012070, as well as to homologs thereof. The nucleic acid sequences corresponding to pre-miRNAs include mir-2478 under accession number MI0011540 (GUUGGGGGUCGGUGGUGAGCAAGCAGAGACUACAUAGGUUGCCUUGUAU CCCACUUCUGACACCAUGUAUUGUCAAC, SEQ ID NO:42).

The term "prevention", as used herein, relates to the capacity to prevent, minimize or hinder the onset or development of a disease or condition before its onset.

The term "prognosis", as used herein, relates to the prediction of a medical outcome, for example, a poor or good outcome (e.g., likelihood of long-term survival, overall survival, disease-specific survival, progression-free survival or disease-free survival); a negative prognosis, or bad prognosis, or poor outcome, includes a prediction of relapse, disease progression, or mortality; a positive prognosis, or good prognosis, or good outcome, includes stabilization of the disease. As will be understood by those skilled in the art, the prediction, although preferred to be, need not be correct for 100% of the subjects to be diagnosed or evaluated. The term, however, requires that a statistically significant portion of subjects can be identified as having an increased probability of having a given outcome. Whether a subject is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 50%, at least 60%, at least 70%, at least 80%, at least 90% at least 95%. The p-values are, preferably 0.05, 0.025, 0.001 or lower.

Any parameter which is widely accepted for determining prognosis of a patient can be used in the present invention including, without limitation, overall survival time.

The term "reference value" or "reference level", as used herein in the context of the method of the invention, relates to a laboratory value used as a reference for the expression level values/data obtained from samples of subjects to be diagnosed with a disease or disorder, in particular diffuse alveolar damage.

The term "sample" or "biological sample", as used herein, refers to biological material isolated from a subject. The biological sample contains any biological material suitable for detecting RNA, particularly miRNA, and is a material comprising genetic material from the subject. The biological sample can comprise cell and/or non-cell material of the subject. In the present invention, the sample comprises genetic material, e.g., DNA, genomic DNA (gDNA), complementary DNA (cDNA), RNA, heterogeneous nuclear RNA (hnRNA), mRNA, etc., from the subject under study. In a particular embodiment, the genetic material is RNA. In a preferred embodiment the RNA is miRNA. The sample can be isolated from any suitable tissue or biological fluid such as, for example blood, saliva, plasma, serum, urine, cerebrospinal liquid (CSF), feces, a surgical specimen, a specimen obtained from a biopsy, and a tissue sample embedded in paraffin. Methods for isolating samples are well known to those skilled in the art. In particular, methods for obtaining a sample from a biopsy include gross apportioning of a mass, or micro-dissection or other art-known cell-separation methods. In order to simplify conservation and handling of the samples, these can be formalin-fixed and paraffin-embedded or first frozen and then embedded in a cryosolidifiable medium, such as OCT-Compound, through immersion in a highly cryogenic medium that allows rapid freeze. In a particular embodiment, the sample from the subject according to the methods of the present invention is selected from the group consisting of blood, serum, plasma, bronchoalveolar lavage, saliva, urine and a tissue sample.

The term "subject" or "individual" or "animal" or "patient" or "mammal," is meant any subject, particularly a mammalian subject, for whom diagnosis, prognosis, or therapy is desired. Mammalian subjects include humans, domestic animals, farm animals, and zoo, sports, or pet animals such as dogs, cats, guinea pigs, rabbits, rats, mice, horses, cattle, cows, and so on. In a preferred embodiment of the invention, the subject is a mammal. In a more preferred embodiment of the invention, the subject is a human.

The term "tiny LNA" refers to a short, e.g., 6, 7, 8, 9, 10, 11 or 12-mer oligonucleotide that is comprised entirely of locked nucleic acid monomers.

The term "treatment", as used herein, refers to both therapeutic measures and prophylactic or preventive measures, wherein the object is to prevent or slow down (lessen) an undesired physiological change or disorder, such as DAD. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

### 2. Diffuse alveolar damage diagnostic method of the invention

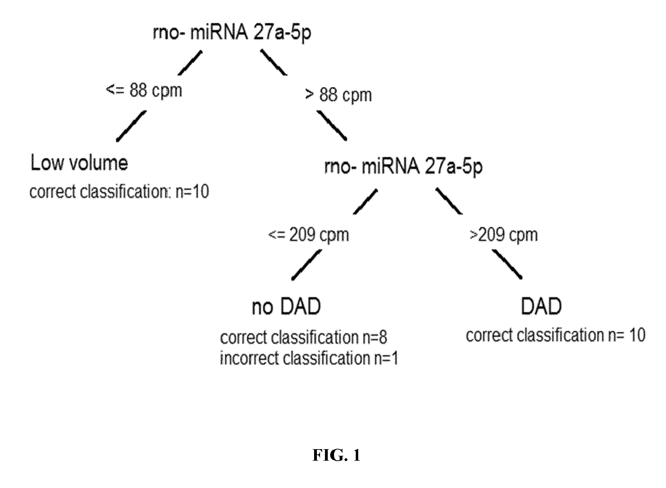

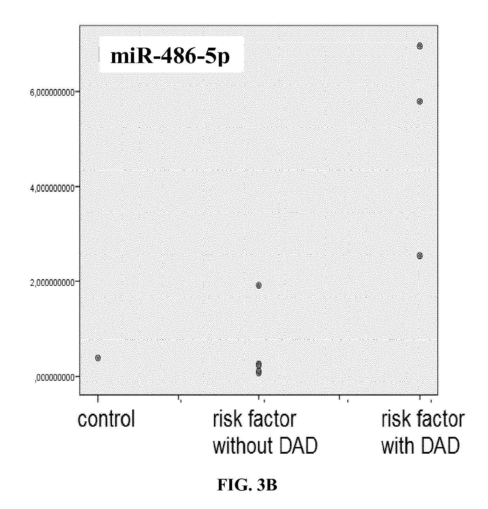

The authors of the present invention have identified a group of 17 miRNAs which are differentially expressed in animals ventilated with low tidal volume, animals ventilated with high tidal volume that do not develop DAD, and animals ventilated with high tidal volume that developed DAD (see Example 1 in the present application). From this group of miRNAs, miR-27a-5p and miR-486-5p showed particularly high sensitivity and specificity in DAD diagnosis (see Example 1 and Figures 1, 2 and 3A in the application). Both miRNAs were also differentially expressed in human lung samples from subjects undergoing DAD (see Example 1, Figure 3B in the application).

Thus, in an aspect, the present invention relates to an *in vitro* method for the diagnosis of diffuse alveolar damage (DAD) in a subject that comprises
(i) determining the level of expression of at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof in a sample from the subject,
(ii) comparing the level of expression of said at least one miRNA to a reference value, and
(iii) correlating a variation in the expression level of said at least one miRNA respect to its reference value with the diagnosis of DAD.

Thus, in a first step, the DAD diagnostic method of the invention comprises determining the level of expression of at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof in a sample from the subject whose diagnosis is to be determined.

A list of the miRNA according to the invention and sequences thereof is shown in Table 1.

**Table 1. miRNAs, accession numbers according to miRBase database (release 21, June 2014) and sequences thereof**

| miRNA | miRBase accession number | Sequence | SEQ ID NO |
|---|---|---|---|
| miR-27a-5p | MIMAT0004501 (hsa) | AGGGCUUAGCUGCUUGUGAGCA | 1 |
| | MIMAT0004633 (mmu) | (hsa, mmu, rno) | |
| | MIMAT0004715 (rno) | | |
| miR-486-5p | MIMAT0002177 (hsa) | UCCUGUACUGAGCUGCCCCGAG (hsa, mmu) | 2 |
| | MIMAT0003130 (mmu) | | |
| miR-2478 | MIMAT0012070 (bta) | GUAUCCCACUUCUGACACCA (bta) | 3 |
| miR-466i-5p | MIMAT0017325 (mmu) | UGUGUGUGUGUGUGUGUGUG (mmu) | 4 |
| miR-125b-1-3p | MIMAT0004592 (hsa) | ACGGGUUAGGCUCUUGGGAGCU (hsa, mmu, rno) | 5 |
| | MIMAT0004669 (mmu) | | |
| | MIMAT0004730 (rno) | | |
| miR-1298 | MIMAT0005800 (hsa) | UUCAUUCGGCUGUCCAGAUGUA (hsa, mmu, rno) | 6 |
| | MIMAT0014809 (mmu) | | |
| | MIMAT0025060 (rno) | | |
| miR-132-5p | MIMAT0004594 (hsa) | ACCGUGGCUUUCGAUUGUUACU (hsa, rno) | 7 |
| | MIMAT0017123 (rno) | | |
| | MIMAT0016984 (mmu) | AACCGUGGCUUUCGAUUGUUAC (mmu) | 8 |
| miR-133a-3p | MIMAT0000427 (hsa) | UUUGGUCCCCUUCAACCAGCUG (hsa, mmu, rno) | 9 |
| | MIMAT0000145 (mmu) | | |
| | MIMAT0000839 (rno) | | |
| miR-200a-3p | MIMAT0000682 (hsa) | UAACACUGUCUGGUAACGAUGU (hsa, mmu, rno) | 10 |
| | MIMAT0000519 (mmu) | | |
| | MIMAT0000874 (rno) | | |
| miR-208a-3p | MIMAT0000241 (hsa) | AUAAGACGAGCAAAAAGCUUGU (hsa, mmu) | 11 |
| | MIMAT0000520 (mmu) | | |
| | MIMAT0000880 (rno) | AUAAGACGAGCAAAAAGC (rno) | 12 |
| miR-21-3p | MIMAT0004494 (hsa) | CAACACCAGUCGAUGGGCUGU (hsa) | 13 |
| | MIMAT0004628 (mmu) | CAACAGCAGUCGAUGGGCUGUC (mmu, rno) | 14 |
| | MIMAT0004711 (rno) | | |
| miR-34a-5p | MIMAT0000255 (hsa) | UGGCAGUGUCUUAGCUGGUUGU (hsa, mmu, rno) | 15 |
| | MIMAT0000542 (mmu) | | |
| | MIMAT0000815 (rno) | | |
| miR-375-3p | MIMAT0000739 (mmu) | UUUGUUCGUUCGGCUCGCGUGA (mmu, rno) | 16 |
| | MIMAT0005307 (rno) | | |
| miR-434-5p | MIMAT0001421 (mmu) | GCUCGACUCAUGGUUUGAACCA (mmu) | 17 |
| | MIMAT0017307 (rno) | AGCUCGACUCAUGGUUUGAACC A (rno) | 18 |
| miR-490-3p | MIMAT0002806 (hsa) | CAACCUGGAGGACUCCAUGCUG (hsa, mmu, rno) | 19 |
| | MIMAT0003780 (mmu) | | |
| | MIMAT0012823 (rno) | | |
| miR-509-5p | MIMAT0004779 (hsa) | UACUGCAGACAGUGGCAAUCA (hsa) | 20 |
| | MIMAT0004890 (mmu) | UACUCCAGAAUGUGGCAAUCAU (mmu) | 21 |
| | MIMAT0024849 (rno) | UACUCCAGAAUAUGGCAAUCAU G (rno) | 22 |
| miR-879-5p | MIMAT0005287 (rno) | AGAGGCUUAUAGCUCUAAGCC (rno, mmu) | 23 |
| | MIMAT0004842 (mmu) | | |

| | | | |
|---|---|---|---|
| hsa: *Homo sapiens;* bta: *Bos taurus;* mmu: *Mus musculus;* rno: *Rattus norvegicus;* sha: *Sarcophilus harrisii* | | | |

Similarly, the first step of the DAD diagnostic method of the invention comprises as well determining the level of expression of at least one variant of the miRNAs above.

According to the invention, variants of any of said at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, as described above can also be determined in a sample from the subject whose diagnosis is to be determined according to the DAD diagnostic method of the invention. Said variants have a nucleic acid sequence which is at least 70 percent, at least 80 percent, at least 90 percent, at least 95 percent, at least 97, at least 98 percent or at least 99 percent identical to the sequence of said at least one miRNA, provided that these variant sequences also encode a functional miRNA.

The percentage of sequence identity, or percentage homology, among nucleotide sequences is determined by comparing two optimally aligned sequences over a comparison window, wherein the portion of the nucleotide sequence in the comparison window may comprise additions or deletions (i.e., gaps) as compared to the reference sequence (which does not comprise additions or deletions) for optimal alignment of the two sequences. The percentage may be calculated by determining the number of positions at which the identical nucleic acid base occurs in both sequences to yield the number of matched positions, dividing the number of matched positions by the total number of positions in the window of comparison and multiplying the result by 100 to yield the percentage of sequence identity. Alternatively, the percentage may be calculated by determining the number of positions at which either the identical nucleotide occurs in both sequences or a nucleotide is aligned with a gap to yield the number of matched positions, dividing the number of matched positions by the total number of positions in the window of comparison and multiplying the result by 100 to yield the percentage of sequence identity. Those of skill in the art appreciate that there are many established algorithms available to align two sequences. Optimal alignment of sequences for comparison can be conducted, e.g., by the local homology algorithm of Smith and Waterman, 1981, Adv. Appl. Math. 2:482, by the homology alignment algorithm of Needleman and Wunsch, 1970, J. MoI. Biol. 48:443, by the search for similarity method of Pearson and Lipman, 1988, Proc. Natl. Acad. ScL USA 85:2444, by computerized implementations of these algorithms {e.g., GAP, BESTFIT, FASTA, and TFASTA in the GCG Wisconsin Software Package), or by visual inspection (see generally, Current Protocols in Molecular Biology, F. M. Ausubel et al., eds., Greene Publishing Associates, Inc. and John Wiley and Sons, Inc., (1995 Supplement)). Examples of algorithms that are suitable for determining percent sequence identity and sequence similarity are the BLAST and BLAST 2.0 algorithms, which are described in Altschul et al., 1990, J. MoI. Biol. 215: 403-410 and Altschul et al., 1977, Nucleic Acids Res. 3389-3402, respectively as well as the BLASTP algorithm (BLAST Manual, Altschul, S. et al., NCBI NLM NIH Bethesda, Md. 20894, Altschul, S., et al., J., 1990, Mol. Biol. 215:403-410). Software for performing BLAST analyses is publicly available through the National Center for Biotechnology Information website. This algorithm involves first identifying high scoring sequence pairs (HSPs) by identifying short words of length W in the query sequence, which either match or satisfy some positive-valued threshold score T when aligned with a word of the same length in a database sequence. T is referred to as, the neighborhood word score threshold (Altschul et al, *supra*). These initial neighbourhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4, and a comparison of both strands (see Henikoff and Henikoff, 1989, Proc Natl Acad Sci USA 89:10915). Exemplary determination of sequence alignment and percent sequence identity can employ the BESTFIT or GAP programs in the GCG Wisconsin Software package (Accelrys, Madison WI), using default parameters provided, or the ClustalW multiple alignment program (available from the European Bioinformatics Institute, Cambridge, UK), using, in some embodiments, the parameters below. "Comparison window" refers to a conceptual segment of at least about 20 contiguous nucleotide positions or amino acids residues wherein a sequence may be compared to a reference sequence of at least 20 contiguous nucleotides or amino acids and wherein the portion of the sequence in the comparison window may comprise additions or deletions (i.e., gaps) of 20 percent or less as compared to the reference sequence (which does not comprise additions or deletions) for optimal alignment of the two sequences. The comparison window can be longer than 20 contiguous residues, and includes, optionally 30, 40, 50, 100, or longer windows

In a particular embodiment, the DAD diagnostic method comprises determining the level of expression of one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, and miR-879-5p in a sample from a subject whose diagnosis is to be determined.

In a more particular embodiment, the diagnostic method comprises determining the level of expression of miR-27a-5p in a sample from a subject whose diagnosis is to be determined.

In a more particular embodiment, the DAD diagnostic method comprises determining the level of expression of miR-486-5p in a sample from a subject whose diagnosis is to be determined.

In a particular embodiment, the DAD diagnostic method comprises determining the level of expression of two miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, and miR-879-5p in a sample from a subject whose diagnosis is to be determined.

In a more particular embodiment, the DAD diagnostic method comprises determining the level of expression of miR-27a-5p and of miR-486-5p in a sample from a subject whose diagnosis is to be determined.

In a particular embodiment, the DAD diagnostic method comprises determining the level of expression of at least three, at least four, at least five, at least six, at least seven, at least eight, at least nine, at least ten, at least eleven, at least twelve, at least thirteen, at least fourteen, at least fifteen, at least sixteen, or at least seventeen miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, and miR-879-5p in a sample from a subject whose diagnosis is to be determined.

In a particular embodiment, the DAD diagnostic method comprises determining the level of expression of all miRNA miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, and miR-879-5p in a sample from a subject whose diagnosis is to be determined.

Before analyzing the sample, it will often be desirable to perform one or more preparation operations upon said sample aimed at separating the molecule to be determined (RNA, in particular miRNA) from other molecules found in the sample. Typically, these sample preparation operations include manipulations such as concentration, suspension, extraction of intracellular material, e.g., nucleic acids from tissue/whole cell samples and the like, amplification of nucleic acids, fragmentation, transcription, labeling and/or extension reactions. Nucleic acids, especially RNA can be isolated using any techniques known in the art. There are two main methods for isolating RNA: (i) phenol-based extraction and (ii) silica matrix or glass fiber filter (GFF)-based binding. Phenol-based reagents contain a combination of denaturants and RNase inhibitors for cell and tissue disruption and subsequent separation of RNA from contaminants. Phenol-based isolation procedures can recover RNA species in the 10-200-nucleotide range e.g., miRNAs, 5S rRNA, 5.8S rRNA, and U1 snRNA. If a sample of "total" RNA was purified by the popular silica matrix column or GFF procedure, it may be depleted in small RNAs. Extraction procedures such as those using Trizol or TriReagent, however will purify all RNAs, large and small, and are the recommended methods for isolating total RNA from biological samples that will contain miRNAs/siRNAs. These methods are typically well known by a person skilled in the art. There are also commercial kits available for miRNA purification including, without limitation, miRNeasy Mini kit from Qiagen, miRNA isolation kits from Life Technologies, mirPremier microRNA isolation kit from Sigma-Aldrich and High Pure miRNA isolation kit from Roche.

The level of expression of at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof is determined in a sample from a subject, wherein said sample is any sample comprising nucleic acids comprising, without limitation, a cell sample (i.e. a lung cell sample), a tissue sample (i.e. a lung tissue sample), a bronchoalveolar lavage sample, a serum sample, a plasma sample, a blood sample, a saliva sample and a urine sample. The sample can be obtained by any conventional method depending on the sample to be analyzed. Methods for obtaining said samples are routinary and known by the skilled person. In a particular embodiment, the sample is a lung tissue sample. Lung tissue samples can be obtained by methods known by the skilled person, including biopsy, surgery and autopsy.

The expression level can be determined using RNA obtained from a formalin-fixed, paraffin-embedded tissue sample or frozen lung sample. RNA may be isolated from an archival pathological sample or biopsy sample which is first deparaffinized. An exemplary deparaffinization method involves washing the paraffinized sample with an organic solvent, such as xylene. Deparaffinized samples can be rehydrated with an aqueous solution of a lower alcohol. Suitable lower alcohols, for example, include methanol, ethanol, propanols and butanols. Deparaffinized samples may be rehydrated with successive washes with lower alcoholic solutions of decreasing concentration, for example. Alternatively, the sample is simultaneously deparaffinized and rehydrated. The sample is then lysed and RNA is extracted from the sample. Samples can be also obtained from fresh tumor tissue such as a resected tumor.

The level of expression of at least one miRNA according to the invention selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof can be determined by any suitable method including generic methods for the detection and quantification of nucleic acids especially RNA, optimized methods for the detection and quantification of small RNA species, as both mature and precursor miRNAs fall into this category, as well as specially designed methods for the detection and quantification of miRNA species. Illustrative, non-limitative, examples of methods which can be used include:
- multiplex and/or singleplex real-time RT-PCR (reagents available from, e.g., Applied Biosystems and System Biosciences), including quantitative real time reverse transcriptase PCR (qRT-PCR),
- single-molecule detection,
- bead-based flow cytometric methods, and
- assays using arrays of nucleic acids.

In a particular embodiment, determination of the levels of RNA, in particular the levels of miRNA, can be carried out by any method known in the art such as qPCR, northern blot, RNA dot blot, TaqMan, tag based methods such as serial analysis of gene expression (SAGE) including variants such as LongSAGE and SuperSAGE, microarrays. Determination of the mRNA levels can also be carried out by fluorescence in situ hybridization (FISH). The detection can be carried out in individual samples or in tissue microarrays.

In a more particular embodiment, the expression levels of at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof in a sample from the subject to be diagnosed with DAD is determined by real-time PCR.

In order to normalize the values of expression of said at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof among the different samples, it is possible to compare the expression levels of said at least miRNA of interest in the test samples with the expression of a control RNA. Thus, in the context of the invention, the term "normalized" expression level refers to the expression level of a miRNA, particularly a miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof relative to the expression level of a single reference gene or control RNA, or a particular set of reference or control RNAs. A "control RNA" as used herein, relates to RNA whose expression level does not change or changes only in limited amounts in samples under analysis with respect to control samples. Preferably, the control RNA is mRNA derived from housekeeping genes and which code for proteins which are constitutively expressed and carry out essential cellular functions. Preferred housekeeping genes for use in the present invention include β-2-microglobulin, ubiquitin, ribosomal protein 18S, cyclophilin, IPO8, HPRT, GAPDH, PSMB4, tubulin, transferrin receptor, tyrosine 3-monooxygenase/tryptophan 5-monooxygenase activation protein (YWHAZ) and β-actin.

In one embodiment, relative miRNA expression quantification is calculated according to the comparative threshold cycle (Ct) method using GAPDH, IPO8, HPRT, β-actin or PSMB4 as an endogenous control and commercial RNA controls as calibrators. Final results are determined according to the formula 2-(ΔCt sample-ΔCt calibrator), where ΔCT values of the calibrator and sample are determined by subtracting the Ct value of the target gene from the value of the control gene.

In a second step of the DAD diagnostic method of the invention, the level of expression of said at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof in a sample from a subject to be diagnosed is compared to a reference value.

The reference value or reference level according to the DAD diagnostic method of the invention can be an absolute value; a relative value; a value that has an upper and/or lower limit; a range of values; an average value; a median value, a mean value, or a value as compared to a particular control or baseline value. A reference value can be based on an individual sample value, such as for example, a value obtained from a sample from the subject being tested, but at an earlier point in time. The reference value can be based on a large number of samples, such as from a population of subjects of the chronological age matched group, or based on a pool of samples including or excluding the sample to be tested. Various considerations are taken into account when determining the reference value of the marker. Among such considerations are the age, weight, sex, general physical condition of the patient and the like. For example, equal amounts of a group of at least 2, at least 10, at least 100 to preferably more than 1000 subjects, preferably classified according to the foregoing considerations, for example according to various age categories, are taken as the reference group.

In a particular embodiment of the DAD diagnostic method of the invention, the reference value of a miRNA selected from group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof is the expression level of said miRNA determined in a sample from a healthy subject or in a sample from a subject who has not been diagnosed with DAD, preferably in a sample from a healthy subject. In another embodiment, the reference value for said miRNA selected from group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof is an average value determined in a pool of healthy subjects or in a pool of healthy subjects who have not been diagnosed with DAD, preferably in a pool of healthy subjects. Said reference value sample is typically obtained by combining equal amounts of samples from a subject population.

In a third step of the diagnostic method of the invention, the variation in the expression level of at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof respect to its corresponding reference value is correlated with the diagnosis of DAD.

Once the reference value for the expression of at least one miRNA selected from group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof is established, the expression levels of said at least miRNA expressed in a sample from a subject to be diagnosed can be compared with this reference value, and thus be assigned a level of "increased" or "decreased". For example, an increase in expression levels above the reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as "increased" expression level. On the other hand, a decrease in expression levels below the reference value of at least 0.9-fold, 0.75-fold, 0.2-fold, 0.1-fold, 0.05-fold, 0.025-fold, 0.02-fold, 0.01-fold, 0.005-fold or even less compared with reference value is considered as "decreased" expression level.

Thus, according to the DAD diagnostic method of the invention,
- if the level of expression of at least one miRNA selected from the group consisting of miR-27a-5p, miR-486-5p, miR-2478, miR-125b-1-3p, miR-132-5p, miR-133a-3p, miR-208a-3p, miR-21-3p, miR-490-3p, and variants thereof is increased respect to its reference value, and/or
- if the level of expression of at least one miRNA selected from the group consisting of miR-1298, miR-200a-3p, miR-34a-5p, miR-375-3p, miR-509-5p, miR-879-5p, miR-466i-5p, miR-434-5p and variants thereof is decreased respect to its reference value,
then the subject is diagnosed with DAD.

In a particular embodiment of the DAD diagnostic method of the invention, the expression level of miR-27a-5p and/or miR-486-5p is determined, and wherein if the level of expression of miR-27a-5p and/or miR-486-5p is increased respect to its reference value, then the subject is diagnosed with DAD.

The person skilled in the art will understand that the prediction may not be correct for 100% of patients under study. However, the expression requires that the prediction method provides correct results for a statistically significant portion of patients. Determination whether the method of the invention provides statistically significant predictions can be carried out by using standard statistical techniques such as the determination of confidence intervals, determination of p value, Student's t-test, Mann-Whitney test. Suitable confidence intervals are at least 50%, at least 60%, at least 70%, at least 80%, at least 90%, at least 95%. p values are, preferably, 0.05, 0.01, 0.005.

### 3. Acute respiratory distress syndrome diagnostic method of the invention

The authors of the present invention have found that the expression levels of some miRNAs, particularly miR-27a-5p, are differentially expressed in patients undergoing ARDS (see Example 2 and Table 5).

Thus, in another aspect, the present invention relates to an *in vitro* for confirming a diagnosis of acute respiratory distress syndrome (ARDS) in a subject suspected to suffer from ARDS that comprises:
(i) determining the level of expression of at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof in a sample from the subject,
(ii) comparing the level of expression of said at least one miRNA to a reference value, and
(iii) correlating a variation in the expression level of said at least one miRNA respect to its reference value with the confirmation of the diagnosis of ARDS in said subject.

Thus, in a first step, the ARDS diagnostic method of the invention comprises determining the level of expression of at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof in a sample from the subject whose ARDS diagnosis is to be confirmed.

According to the invention, variants of any of said at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, and miR-879-5p as described above can also be determined in a sample from the subject whose diagnosis is to be confirmed according to the ARDS diagnostic method of the invention. Said variants have a nucleic acid sequence which is at least 70 percent, at least 80 percent, at least 90 percent, at least 95 percent, at least 97, at least 98 percent or at least 99 percent identical to the sequence of said at least one miRNA, provided that these variant sequences also encode a functional miRNA. Methods to determine the percentage of sequence identity have been described previously in the context of the DAD diagnostic method of the invention and are incorporated herein.

In a particular embodiment, the ARDS diagnostic method comprises determining the level of expression of one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, and miR-879-5p in a sample from a subject whose ARDS diagnosis is to be confirmed.

In a more particular embodiment, the ARDS diagnostic method comprises determining the level of expression of miR-27a-5p in a sample from a subject whose ARDS diagnosis is to be confirmed.

In a more particular embodiment, the ARDS diagnostic method comprises determining the level of expression of miR-486-5p in a sample from a subject whose ARDS diagnosis is to be confirmed.

In a particular embodiment, the ARDS diagnostic method comprises determining the level of expression of two miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, and miR-879-5p in a sample from a subject whose ARDS diagnosis is to be confirmed.

In a more particular embodiment, the ARDS diagnostic method comprises determining the level of expression of miR-27a-5p and of miR-486-5p in a sample from a subject whose diagnosis is to be confirmed.

In a particular embodiment, the ARDS diagnostic method comprises determining the level of expression of at least three, at least four, at least five, at least six, at least seven, at least eight, at least nine, at least ten, at least eleven, at least twelve, at least thirteen, at least fourteen, at least fifteen, at least sixteen, or at least seventeen miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, and miR-879-5p in a sample from a subject whose diagnosis is to be confirmed.

In a particular embodiment, the ARDS diagnostic method comprises determining the level of expression of all miRNA miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, and miR-879-5p in a sample from a subject whose diagnosis is to be confirmed.

Methods for obtaining miRNA from a sample of a subject, for analyzing miRNA expression in a sample from a subject, as well as for normalization of miRNA expression values have been previously described in the context of the DAD diagnostic method of the invention and incorporated herein.

The sample from the subject suspected to suffer from ARDS comprises any sample comprising nucleic acids comprising, without limitation, a cell sample (i.e. a lung cell sample), a tissue sample (i.e. a lung tissue sample), a bronchoalveolar lavage sample, a serum sample, a plasma sample, a blood sample, a saliva sample and a urine sample. The sample can be obtained by any conventional method depending on the sample to be analyzed. Methods for obtaining said samples are routinary and known by the skilled person. In a particular embodiment, the sample is a lung tissue sample.

The subject suspected to suffer from ARDS is a subject showing severe hypoxemia not completely explained from heart failure, bilateral pulmonary infiltrates and a risk factor. ARDS risk factors include, without limitation, sepsis, shock, traumatism, pulmonary contusion, pneumonia, pancreatitis, burning, semi-drowning, pulmonary aspiration, heatstroke, fat embolism, amniotic fluid embolism, drug intoxication, and ischemia/reperfusion injury.

In a second step of the ARDS diagnostic method of the invention, the level of expression of said at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof in a sample from a subject whose ARDS diagnosis is to be confirmed is compared to a reference value.

The reference value or reference level according to the ARDS diagnostic method of the invention can be an absolute value; a relative value; a value that has an upper and/or lower limit; a range of values; an average value; a median value, a mean value, or a value as compared to a particular control or baseline value. A reference value can be based on an individual sample value, such as for example, a value obtained from a sample from the subject being tested, but at an earlier point in time. The reference value can be based on a large number of samples, such as from a population of subjects of the chronological age matched group, or based on a pool of samples including or excluding the sample to be tested. Various considerations are taken into account when determining the reference value of the marker. Among such considerations are the age, weight, sex, general physical condition of the patient and the like. For example, equal amounts of a group of at least 2, at least 10, at least 100 to preferably more than 1000 subjects, preferably classified according to the foregoing considerations, for example according to various age categories, are taken as the reference group.

In a particular embodiment of the ARDS diagnostic method of the invention, the reference value of a miRNA selected from group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof is the expression level of said miRNA determined in a sample from a healthy subject or in a sample from a subject who has not been diagnosed with ARDS, preferably in a sample from a healthy subject. In another embodiment, the reference value for said miRNA selected from group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof is an average value determined in a pool of healthy subjects or in a pool of subjects who have not been diagnosed with ARDS, preferably in a pool of healthy subjects. Said reference value sample is typically obtained by combining equal amounts of samples from a subject population.

In a third step of the diagnostic method of the invention, the variation in the expression level of at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof respect to its corresponding reference value is correlated with the confirmation of the diagnosis of ARDS.

Thus, according to the ARDS diagnostic method of the invention,
- if the level of expression of at least one miRNA selected from the group consisting of miR-27a-5p, miR-486-5p, miR-2478, miR-125b-1-3p, miR-132-5p, miR-133a-3p, miR-208a-3p, miR-21-3p, miR-490-3p, and variants thereof is increased respect to its reference value, and/or
- if the level of expression of at least one miRNA selected from the group consisting of miR-1298, miR-200a-3p, miR-34a-5p, miR-375-3p, miR-509-5p, miR-879-5p, miR-466i-5p, miR-434-5p and variants thereof is decreased respect to its reference value,then the diagnosis of ARDS is confirmed.

Alternatively, according to the ARDS diagnostic method of the invention,
- if the level of expression of at least one miRNA selected from the group consisting of miR-27a-5p, miR-486-5p, miR-2478, miR-125b-1-3p, miR-132-5p, miR-133a-3p, miR-208a-3p, miR-21-3p, miR-490-3p, and variants thereof is decreased respect to its reference value, and/or
- if the level of expression of at least one miRNA selected from the group consisting of miR-1298, miR-200a-3p, miR-34a-5p, miR-375-3p, miR-509-5p, miR-879-5p, miR-466i-5p, miR-434-5p and variants thereof is increased respect to its reference value,
then the diagnosis of ARDS is not confirmed.

The terms "increased expression value" and "decreased expression value" in connection to the level of expression of the miRNA of the invention have been previously described in the context of the DAD diagnostic method of the invention and incorporated herein.

In a particular embodiment of the ARDS diagnostic method of the invention, the expression level of miR-27a-5p and/or miR-486-5p is determined, and wherein
if the level of expression of miR-27a-5p and/or miR-486-5p is increased respect to its reference value, then the diagnosis of ARDS is confirmed,
or, alternatively,
if the level of expression of miR-27a-5p and/or miR-486-5p is decreased respect to its reference value, then the diagnosis of ARDS is not confirmed.

More particularly, the expression level of miR-27a-is determined, and wherein if the level of expression of miR-27a-5p is increased respect to its reference value, then the diagnosis of ARDS is confirmed, or alternatively, if the level of expression of miR-27a-5p is decreased respect to its reference value, then the diagnosis of ARDS is not confirmed.

The person skilled in the art will understand that the prediction may not be correct for 100% of patients under study. However, the expression requires that the prediction method provides correct results for a statistically significant portion of patients. Determination whether the method of the invention provides statistically significant predictions can be carried out by using standard statistical techniques such as the determination of confidence intervals, determination of p value, Student's t-test, Mann-Whitney test. Suitable confidence intervals are at least 50%, at least 60%, at least 70%, at least 80%, at least 90%, at least 95%. p values are, preferably, 0.05, 0.01, 0.005.

### 4. Prognostic method of the invention

The authors of the present invention have identified miRNAs with prognostic value (i.e. mortality) in patients undergoing mechanical ventilation (see Example 2 and

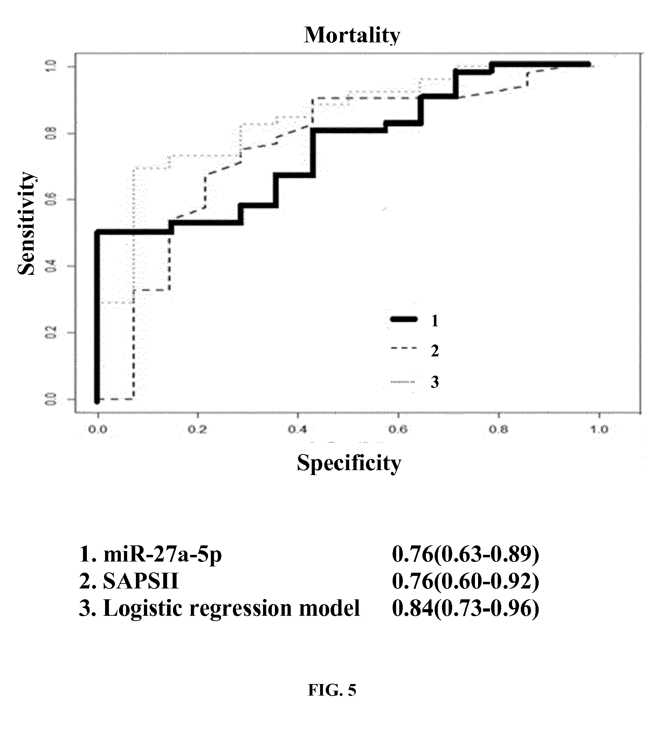

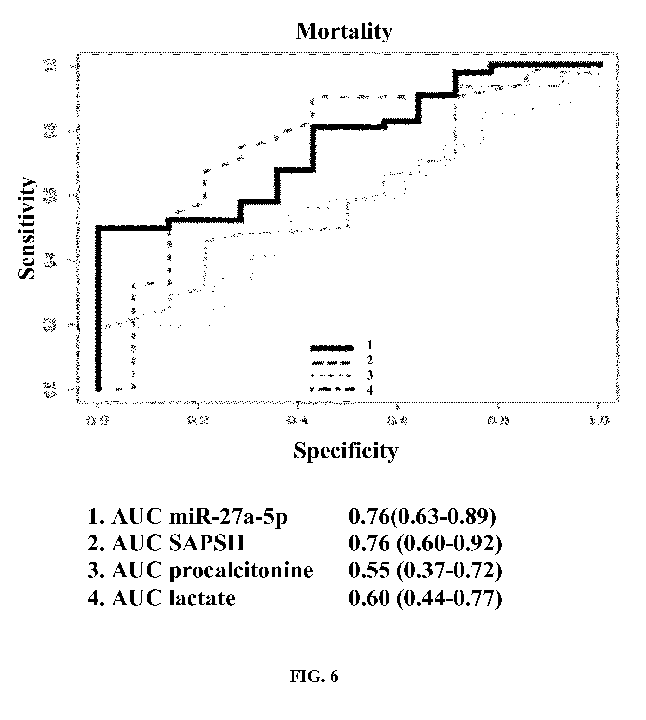

Figures 5 and 6 in the present application). In particular, miR-27a-5p showed a high discriminative capacity in the prognosis of patients undergoing mechanical ventilation.

Thus, in another aspect, the present invention relates to an *in vitro* for determining the prognosis of a subject undergoing mechanical ventilation that comprises
(i) determining the level of expression of at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof in a sample from the subject,
(ii) comparing the level of expression of said at least one miRNA to a reference value, and
(iii) correlating a variation in the expression level of said at least one miRNA respect to its reference value with the prognosis of said subject undergoing chemical ventilation.

Thus, in a first aspect, the prognostic method of the invention comprises determining the level of expression of at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof in a sample from a subject undergoing mechanical ventilation whose prognosis is to be determined.

According to the invention, variants of any of said at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, and miR-879-5p as described above can also be determined in a sample from the subject undergoing mechanical ventilation whose prognosis is to be determined. Said variants have a nucleic acid sequence which is at least 70 percent, at least 80 percent, at least 90 percent, at least 95 percent, at least 97, at least 98 percent or at least 99 percent identical to the sequence of said at least one miRNA, provided that these variant sequences also encode a functional miRNA. Methods to determine the percentage of sequence identity have been described previously in the context of the DAD diagnostic method of the invention and are incorporated herein.

In a particular embodiment, the prognostic method comprises determining the level of expression of one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, and miR-879-5p in a sample from a subject undergoing mechanical ventilation whose prognosis is to be determined.

In a more particular embodiment, the prognostic method comprises determining the level of expression of miR-27a-5p in a sample from a subject undergoing mechanical ventilation whose prognosis is to be determined.

In a more particular embodiment, the prognostic method comprises determining the level of expression of miR-486-5p in a sample from a subject undergoing mechanical ventilation whose prognosis is to be determined.

In a particular embodiment, the prognostic method comprises determining the level of expression of two miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, and miR-879-5p in a sample from a subject undergoing mechanical ventilation whose prognosis is to be determined.

In a more particular embodiment, the prognostic method comprises determining the level of expression of miR-27a-5p and of miR-486-5p in a sample from a subject undergoing mechanical ventilation whose prognosis is to be determined.

In a particular embodiment, the prognostic method comprises determining the level of expression of at least three, at least four, at least five, at least six, at least seven, at least eight, at least nine, at least ten, at least eleven, at least twelve, at least thirteen, at least fourteen, at least fifteen, at least sixteen, or at least seventeen miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, and miR-879-5p in a sample from a subject undergoing mechanical ventilation whose prognosis is to be determined.

In a particular embodiment, the prognostic method comprises determining the level of expression of all miRNA miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, and miR-879-5p in a sample from a subject undergoing mechanical ventilation whose prognosis is to be determined.

Methods for obtaining miRNA from a sample of a subject, for analyzing miRNA expression in a sample from a subject, as well as for normalization of miRNA expression values have been previously described in the context of the DAD diagnostic method of the invention and incorporated herein.

The sample from the subject undergoing mechanical ventilation whose prognosis is to be determined comprises any sample comprising nucleic acids comprising, without limitation, a cell sample (i.e. a lung cell sample), a tissue sample (i.e. a lung tissue sample), a bronchoalveolar lavage sample, a serum sample, a plasma sample, a blood sample, a saliva sample and a urine sample. The sample can be obtained by any conventional method depending on the sample to be analyzed. Methods for obtaining said samples are routinary and known by the skilled person. In a particular embodiment, the sample is a lung tissue sample.

The subject undergoing mechanical ventilation whose prognosis is to be determined is a subject whose spontaneous ventilation is inadequate to maintain life, undergoing ineffective gas exchange in the lungs or suffering from imminent collapse. Common medical indications for mechanical ventilation are known by the skilled person and include, without limitation, severe acute or chronic hypoxemia, mental status impairment, shock and surgery or invasive procedures. acute lung injury including ARDS, apnea with respiratory arrest, chronic obstructive pulmonary disease (COPD), acute respiratory acidosis with partial pressure of carbon dioxide (pCO₂) > 50 mmHg and pH < 7.25), tachypnea, hypoxemia with arterial partial pressure of oxygen (PaO₂) < 55 mm Hg and with supplemental fraction of inspired oxygen (FiO₂) = 1.0, sepsis, shock, congestive heart failure, and neurological diseases such as muscular dystrophy and amyotrophic lateral sclerosis.

In a second step of the diagnostic method of the invention, the level of expression of said at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof in a sample from the subject undergoing mechanical ventilation whose prognosis is to be determined is compared to a reference value.

The reference value or reference level according to the prognostic method of the invention can be an absolute value; a relative value; a value that has an upper and/or lower limit; a range of values; an average value; a median value, a mean value, or a value as compared to a particular control or baseline value. A reference value can be based on an individual sample value, such as for example, a value obtained from a sample from the subject being tested, but at an earlier point in time. The reference value can be based on a large number of samples, such as from a population of subjects of the chronological age matched group, or based on a pool of samples including or excluding the sample to be tested. Various considerations are taken into account when determining the reference value of the marker. Among such considerations are the age, weight, sex, general physical condition of the patient and the like. For example, equal amounts of a group of at least 2, at least 10, at least 100 to preferably more than 1000 subjects, preferably classified according to the foregoing considerations, for example according to various age categories, are taken as the reference group.

In a particular embodiment of the prognostic method of the invention, the reference value of a miRNA selected from group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof is the expression level of said miRNA determined in a sample from a healthy subject or in a sample from a subject who is not undergoing mechanical ventilation, preferably in a sample from a healthy subject. In another embodiment, the reference value for said miRNA selected from group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof is an average value determined in a pool of healthy subjects or in a pool of subjects not undergoing mechanical ventilation, preferably in a pool of healthy subjects. Said reference value sample is typically obtained by combining equal amounts of samples from a subject population.

In a third step of the prognostic method of the invention, the variation in the expression level of at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof respect to its corresponding reference value is correlated with the prognosis of the subject undergoing mechanical ventilation.

Thus, according to the prognostic method of the invention,
- if the level of expression of at least one miRNA selected from the group consisting of miR-27a-5p, miR-486-5p, miR-2478, miR-125b-1-3p, miR-132-5p, miR-133a-3p, miR-208a-3p, miR-21-3p, miR-490-3p, and variants thereof is increased respect to its reference value, and/or
- if the level of expression of at least one miRNA selected from the group consisting of miR-1298, miR-200a-3p, miR-34a-5p, miR-375-3p, miR-509-5p, miR-879-5p, miR-466i-5p, miR-434-5p and variants thereof is decreased respect to its reference value,
it is indicative of a bad prognosis of the subject undergoing mechanical ventilation,

Alternatively, according to the prognostic method of the invention,
- if the level of expression of at least one miRNA selected from the group consisting of miR-27a-5p, miR-486-5p, miR-2478, miR-125b-1-3p, miR-132-5p, miR-133a-3p, miR-208a-3p, miR-21-3p, miR-490-3p, and variants thereof is decreased respect to its reference value, and/or
- if the level of expression of at least one miRNA selected from the group consisting of miR-1298, miR-200a-3p, miR-34a-5p, miR-375-3p, miR-509-5p, miR-879-5p, miR-466i-5p, miR-434-5p and variants thereof is increased respect to its reference value,
it is indicative of a good prognosis of the subject undergoing mechanical ventilation.

The terms "increased expression value" and "decreased expression value" in connection to the level of expression of the miRNA of the invention have been previously described in the context of the DAD diagnostic method of the invention and incorporated herein.

In a particular embodiment of the prognostic method of the invention, the expression level of miR-27a-5p and/or miR-486-5p is determined, and wherein
if the level of expression of miR-27a-5p and/or miR-486-5p is increased respect to its reference value, it is indicative of a bad prognosis of the subject undergoing mechanical ventilation,
or, alternatively,
if the level of expression of miR-27a-5p and/or miR-486-5p is decreased respect to its reference value, it is indicative of a good prognosis of the subject undergoing mechanical ventilation.

More particularly, the expression level of miR-27a-is determined, and wherein if the level of expression of miR-27a-5p is increased respect to its reference value, then the diagnosis of ARDS is confirmed, or alternatively, if the level of expression of miR-27a-5p is decreased respect to its reference value, then the diagnosis of ARDS is not confirmed.

The person skilled in the art will understand that the prediction may not be correct for 100% of patients under study. However, the expression requires that the prediction method provides correct results for a statistically significant portion of patients. Determination whether the method of the invention provides statistically significant predictions can be carried out by using standard statistical techniques such as the determination of confidence intervals, determination of p value, Student's t-test, Mann-Whitney test. Suitable confidence intervals are at least 50%, at least 60%, at least 70%, at least 80%, at least 90%, at least 95%. p values are, preferably, 0.05, 0.01, 0.005.

### 5. Agents for use of the invention

In a further aspect, the invention relates to an agent for use in the prevention and/or treatment of DAD in a subject selected from:
- an agent inhibiting the expression of a miRNA selected from the group consisting of miR-27a-5p, miR-486-5p, miR-2478, miR-125b-1-3p, miR-132-5p, miR-133a-3p, miR-208a-3p, miR-21-3p and miR-490-3p, and
- an agent inducing the expression of a miRNA selected from the group consisting of miR-1298, miR-200a-3p, miR-34a-5p, miR-375-3p, miR-509-5p, miR-879-5p, miR-466i-5p and miR-434-5p.

Alternatively, the invention relates to the use of an agent selected from
- an agent inhibiting the expression of a miRNA selected from the group consisting of miR-27a-5p, miR-486-5p, miR-2478, miR-125b-1-3p, miR-132-5p, miR-133a-3p, miR-208a-3p, miR-21-3p and miR-490-3p, and
- an agent inducing the expression of a miRNA selected from the group consisting of miR-1298, miR-200a-3p, miR-34a-5p, miR-375-3p, miR-509-5p, miR-879-5p, miR-466i-5p and miR-434-5p,
for the manufacture of a medicament for the prevention and/or treatment of DAD in a subject in need thereof.

Alternatively, the invention relates to a method for the prevention and/or treatment of DAD in a subject in need thereof that comprises the administration of a therapeutically effective amount to said subject of an agent selected from:
- an agent inhibiting the expression of a miRNA selected from the group consisting of miR-27a-5p, miR-486-5p, miR-2478, miR-125b-1-3p, miR-132-5p, miR-133a-3p, miR-208a-3p, miR-21-3p and miR-490-3p, and
- an agent inducing the expression of a miRNA selected from the group consisting of miR-1298, miR-200a-3p, miR-34a-5p, miR-375-3p, miR-509-5p, miR-879-5p, miR-466i-5p and miR-434-5p.

### Agents inhibiting miRNA expression for use in the prevention and/or treatment of DAD

The invention relates to an agent inhibiting the expression of a miRNA selected from the group consisting of miR-27a-5p, miR-486-5p, miR-2478, miR-125b-1-3p, miR-132-5p, miR-133a-3p, miR-208a-3p, miR-21-3p and miR-490-3p, for use in the prevention and/or treatment of DAD in a subject.

Agents inhibiting miRNA expression, also known as "anti-miRNA agents", are known by the skilled person and include agents inhibiting both miRNA and/or corresponding pre-miRNA expression. Agents inhibiting miRNA expression according to the invention include, without limitation, small molecules, nucleic acids, nucleic acid analogues, aptamers, ribozymes, peptides, proteins, antibodies, as well as variants and fragments thereof.

In a particular embodiment, the agent inhibiting miRNA expression for use according to the invention is a nucleic acid agent selected from the group comprising DNA, RNA (small inhibitory RNA or RNAi, siRNA, microRNA, shRNA, miRNA), a nucleic acid analogue, a peptide nucleic acid (PNA), a pseudo-complementary PNA (pcPNA), a locked nucleic acid (LNA), and any analogue or variant thereof effective in gene silencing.

In a particular embodiment, the agent inhibiting miRNA expression for use according to the invention is an antagomir (also known as anti-miR or blockmir, a small synthetic RNA that is substantially complementary to the specific miRNA target with either mispairing at the cleavage site of Ago2 or some sort of base modification to inhibit Ago2 cleavage), a fully modified 2'-O-methoxyethyl (2'-MOE) oligonucleotide, a 2'-F/MOE mixmer, a LNA/DNA mixmer, a tiny LNA or a combination thereof, which are complementary to, or complementary in part, to the miRNA seed sequence of the miRNA according to the invention, as previously described.

The term "tiny LNA" refers to a short, e.g., 6, 7, 8, 9, 10, 11 or 12-mer oligonucleotide that is comprised entirely of locked nucleic acid monomers. Tiny LNAs are described by Obad (Obad S et al. 2011 Nature Genetics 43(4): 371-380). In some embodiments, the tiny LNA comprises phosphorothioate inter-sugar linkages at all positions. In some embodiments, the tiny LNA is 8 nucleotides in length and comprises phosphorothioate inter-sugar linkages at all positions. In some embodiments, the tiny LNA comprises a nucleotide sequence complementary to the seed sequence of the miRNA according to the invention as described above.

Agents inhibiting the expression of at least one miRNA selected from the group consisting of miR-27a-5p, miR-486-5p, miR-2478; miR-5109, miR-125b-1-3p, miR-132-5p, miR-133a-3p, miR-208a-3p, miR-21-3p and miR-490-3p for use according to the invention include as well commercially available inhibitors including, without limitation, inhibitors of miR-27a-5p such as the miRNA inhibitor against hsa-miR-27a-5p from Tebu-Bio (catalog No. HmiR-AN0358-AM03-B), anti-hsa-miR-27a-5p miScript miRNA inhibitor from Qiagen (catalog No. MIN0004501), and hsa-mir-27a-5p miRCURY LNA™ Power microRNA inhibitor from Exiqon (catalog No. 4103529-101).

### Agents inducing miRNA expression for use in the prevention and/or treatment of DAD

The invention relates as well to an agent inducing the expression of a miRNA selected from the group consisting of miR-1298, miR-200a-3p, miR-34a-5p, miR-375-3p, miR-509-5p, miR-879-5p, miR-466i-5p and miR-434-5p for use in the prevention and/or treatment of DAD in a subject.

Agents inducing miRNA expression for use according to the invention are selected from the group consisting of said miRNA, a variant of said miRNA, an agent inducing the expression of said miRNA, and an agent inducing the expression of a variant of said miRNA. Thus,
- the agent inducing the expression of miR-1298 is selected from the group consisting of miR-1298, a miR-1298 variant, and agent inducing the expression of miR-1298 and an agent inducing the expression of a variant of miR-1298,
- the agent inducing the expression of miR-200a-3p is selected from the group consisting of miR-200a-3p, a miR-200a-3p variant, and agent inducing the expression of miR-200a-3p and an agent inducing the expression of a variant of miR-200a-3p,
- the agent inducing the expression of miR-34a-5p is selected from the group consisting of miR-34a-5p, a miR-34a-5p variant, and agent inducing the expression of miR-34a-5p and an agent inducing the expression of a variant of miR-34a-5p,
- the agent inducing the expression of miR-375-3p is selected from the group consisting of miR-375-3p, a miR-375-3p variant, and agent inducing the expression of miR-375-3p and an agent inducing the expression of a variant of miR-375-3p,
- the agent inducing the expression of miR-509-5p is selected from the group consisting of miR-509-5p, a miR-509-5p variant, and agent inducing the expression of miR-509-5p and an agent inducing the expression of a variant of miR-509-5p,
- the agent inducing the expression of miR-879-5p is selected from the group consisting of miR-879-5p, a miR-879-5p variant, and agent inducing the expression of miR-879-5p and an agent inducing the expression of a variant of miR-879-5p,
- the agent inducing the expression of miR-466i-5p is selected from the group consisting of miR-466i-5p, a miR-466i-5p variant, and agent inducing the expression of miR-466i-5p and an agent inducing the expression of a variant of miR-466i-5p, and
- the agent inducing the expression of miR-434-5p is selected from the group consisting of miR-434-5p, a miR-434-5p variant, and agent inducing the expression of miR-434-5p and an agent inducing the expression of a variant of miR-434-5p.

miRNAs miR-1298, miR-200a-3p, miR-34a-5p, miR-375-3p, miR-509-5p, miR-879-5p, miR-466i-5p and miR 434-5p, as well as variants thereof, have been previously described.

Agents inducing the expression of a miRNA selected from the group consisting of miR-1298, miR-200a-3p, miR-34a-5p, miR-375-3p, miR-509-5p, miR-879-5p, miR-466i-5p and miR-434-5p, or of a variant thereof, for use according to the invention comprise any molecule capable of increasing the expression of said miRNA or of a variant thereof. Suitable methods for determining whether an agent is capable of increasing or up-regulating the levels of a miRNA (or a variant thereof) include, without limitation, standard assays for determining mRNA expression levels such as qPCR, RT-PCR, RNA protection analysis, Northern blot, RNA dot blot, *in situ* hybridization, microarray technology, tag based methods such as serial analysis of gene expression (SAGE) including variants such as LongSAGE and SuperSAGE, microarrays, fluorescence in situ hybridization (FISH), including variants such as Flow-FISH, qFiSH and double fusion FISH (D-FISH), and the like.

In an embodiment of the invention, agents inducing expression of a miRNA selected from the group consisting of miR-1298, miR-200a-3p, miR-34a-5p, miR-375-3p, miR-509-5p, and miR-879-5p, miR-466i-5p and miR-434-5p for use according to the invention are selected from the group consisting of the pre-miRNA corresponding to said miRNA, a polynucleotide encoding said miRNA and a polynucleotide encoding the pre-miRNA corresponding to said miRNA.

### Agents inducing the expression of miRNA: pre-miRNA

In an embodiment, the agent inducing the expression of a miRNA selected from the group consisting of miR-1298, miR-200a-3p, miR-34a-5p, miR-375-3p, miR-509-5p, miR-879-5p, miR-466i-5p and miR-434-5p for use according to the invention is the pre-miRNA corresponding to said miRNA.

Thus, in a particular embodiment:
- the agent inducing the expression of miR-1298 is a pre-mir-1298 including, without limitation, human mir-1298 (miRBase release 21 accession number MI0003938), mouse mir-1298 (miRBase accession number MI0004300) and rat mir-1298 (miRBase release 21 accession number MI0021845)
- the agent inducing the expression of miR-200a-3p is a pre-mir-200a including, without limitation, human mir-200a (miRBase release 21 accession number MI0000737), mouse mir-200a (miRBase accession number MI0000554), and rat mir-200a (miRBase accession number MI0000943).
- the agent inducing the expression of miR-34a-5p is a pre-mir-34a including, without limitation, human miR-34a (miRBase release 21 accession number MI0000268), mouse miR-34a (miRBase accession number MI0000584) and rat miR-34a.
- the agent inducing the expression of miR-375-3p is a pre-mir-375 including, without limitation, human mir-375 (miRBase release 21 accession number MI0000783), mouse mir-375 (miRBase accession number MI0000792), and rat mir-375.
- the agent inducing the expression of miR-509-5p is a pre-mir-509 including, without limitation, human mir-509-1 (miRBase release 21 accession number MI0003196), mouse mir-509, and rat mir-509.
- the agent inducing the expression of miR-879-5p is a pre-mir-879 including, without limitation, human mir-879, mouse mir-879 (miRBase release 21 accession number MI0005472), and rat mir-879.
- the agent inducing the expression of miR-466i-5p is a pre-mir-466i including, without limitation, human mir-466i, mouse mir-466i (miRBase release 21 accession number MI0006282), and rat mir-466i.
- the agent inducing the expression of miR-434-5p is a pre-mir-434 including, without limitation, human mir-434, mouse mir-434 (miRBase release 21 accession number MI0001526), and rat mir-434.

Additionally, synthetic exemplary pre-miRNA for use according to the invention include, without limitation, the Pre-miR^{™} miRNA precursors that are synthesized on demand by Ambion.

It will, of course, be understood that variants of said pre-miRNA can also be used in the context of the present invention. Said variants have a nucleic acid sequence which is at least 70 percent, at least 80 percent, at least 90 percent, at least 95 percent, at least 97, at least 98 percent or at least 99 percent identical to the sequence of a miRNA selected from the group consisting of miR-1298, miR-200a-3p, miR-34a-5p, miR-375-3p, miR-509-5p, and miR-879-5p, provided that these variant sequences are processed to a functional mRNA or a variant thereof.

Other variants which can be used in the method according to the present invention include polynucleotides which hybridize to the above polynucleotides, preferably, under stringent hybridization conditions provided that the variants retain the properties of miR-1298, miR-200a-3p, miR-34a-5p, miR-375-3p, miR-509-5p, or miR-879-5p, as set forth above.

Other variants include polynucleotides which are identical to pre-miRNA mir-1298, mir-200a-3p, mir-34a-5p, mir-375-3p, mir-509-5p, or mir-879-5p in the region which corresponds, respectively, to the mature miR-1298, miR-200a-3p, miR-34a-5p, miR-375-3p, miR-509-5p, miR-879-5p, or but which differ in the region that forms in the 5' and 3' region which form the stem-loop and which is removed during processing to provide the mature miRNA.

### Agents inducing the expression of miRNA: polynucleotides encoding miRNA or encoding corresponding pre-miRNA.

In another embodiment, the agent inducing expression of miR-1298, miR-200a-3p, miR-34a-5p, miR-375-3p, miR-509-5p, miR-879-5p, miR-466i-5p or miR-434-5p for use according to the invention is a polynucleotide encoding, respectively, miR-1298, miR-200a-3p, miR-34a-5p, miR-375-3p, miR-509-5p, miR-879-5p, miR-466i-5p or miR-434-5p, preferably human miRNA, or a polynucleotide encoding pre-miRNA, respectively, mir-1298, mir-200a, mir-34a, mir-375, mir-509, mir-879, mir-466i or mir-434, preferably human pre-miRNA. In a particular embodiment, said polynucleotides are provided within a vector, preferably within a lentiviral vector.

It will, of course, be understood that variants of said polynucleotide encoding miRNA or pre-miRNA can also be used in the context of the present invention. Said variants have a nucleic acid sequence which is at least 70 percent, at least 80 percent, at least 90 percent, at least 95 percent, at least 97, at least 98 percent or at least 99 percent identical to the sequence of the polynucleotides encoding miR-1298, miR-200a-3p, miR-34a-5p, miR-375-3p, miR-509-5p, miR-879-5p, miR-466i-5p or miR-434-5p, or to the sequence of the polynucleotides encoding mir-1298, mir-200a, mir-34a, mir-375, mir-509, mir-879, mir-466i or mir-434 provided that these variant sequences also encode a pre-miRNA which can be processed into a functional miRNA. Other variants which can be used in the method according to the present invention include polynucleotides which hybridize to the above polynucleotides, preferably, under stringent hybridization conditions provided that the variants retain the properties of a miRNA or of a pre-miRNA as set forth above.

Preferably, the polynucleotide encoding miR-1298, miR-200a-3p, miR-34a-5p, miR-375-3p, miR-509-5p, miR-879-5p, miR-466i-5p, miR-434-5p and/or the polynucleotide encoding mir-1298, mir-200a, mir-34a, mir-375, mir-509, or mir-879, mir-466i and mir-434 further comprises from 200 to 400 base pairs upstream and/or downstream flanking the genomic sequence of said miRNA or pre-miRNA, ensuring that the microRNA expressed from the polynucleotide for use according to the invention will be correctly processed in the cell into mature miRNA.

In a particular embodiment, the polynucleotide encoding miR-1298, miR-200a-3p, miR-34a-5p, miR-375-3p, miR-509-5p, miR-879-5p, miR-466i-5p, miR-434-5p and/or the polynucleotide encoding mir-1298, mir-200a, mir-34a, mir-375, mir-509, mir-879, mir-466i and mir-434 for use according to the invention comprise(s) a transcriptional regulatory sequence. The term "transcriptional regulatory sequence", as used herein, refers to a nucleic acid sequence which is capable of governing the expression of another nucleic acid sequence operatively linked thereto, such as a nucleotide sequence encoding a miRNA or a nucleotide sequence encoding the corresponding pre-miRNA. The transcription control sequence, preferably, is a DNA sequence. The transcriptional control sequence comprises a first promoter and, optionally, a binding site for a transcriptional repressor wherein said first promoter and said binding site for a transcriptional repressor are arranged so that the binding of the transcriptional repressor to said binding site inhibits the transcriptional activity of the promoter.

The term "promoter", as used herein, refers to a DNA sequence that determines the site of transcription initiation for an RNA polymerase. Promoter sequences comprise motifs which are recognized and bound by polypeptides, i.e. transcription factors. The said transcription factors shall upon binding recruit RNA polymerases II, preferably, RNA polymerase I, II or III, more preferably, RNA polymerase II or III, and most preferably, RNA polymerase II. Thereby will be initiated the expression of a nucleic acid operatively linked to the transcription control sequence. It is to be understood that dependent on the type of nucleic acid to be expressed, expression as meant herein may comprise transcription of DNA sequences into RNA polynucleotides (as suitable for, e.g., anti-sense approaches, RNAi approaches or ribozyme approaches) or may comprise transcription of DNA sequences into RNA polynucleotides followed by translation of the said RNA polynucleotides into polypeptides (as suitable for, e.g., gene expression and recombinant polypeptide production approaches). In order to govern expression of a nucleic acid sequence, the transcription control sequence may be located immediately adjacent to the nucleic acid to be expressed, i.e. physically linked to the said nucleic acid at its 5' end. Alternatively, it may be located in physical proximity. In the latter case, however, the sequence must be located so as to allow functional interaction with the nucleic acid to be expressed.

Suitable promoters for use as first promoters include any promoter known in the art. In a preferred embodiment, the first promoter is a promoter functional in mammalian cells. High-level constitutive promoters are preferred for use in the vectors according to the present invention. Examples of such promoters include, without limitation, the retroviral Rous sarcoma virus (RSN) LTR promoter (optionally with the RSV enhancer), the cytomegalovirus (CMV) promoter (optionally with the CMV enhancer), the SN40 promoter, the dihydrofolate reductase promoter, the beta-actin promoter, the beta-actine promoter linked to the enhancer derived from the cytomegalovirus immediate early (IE) promoter, the phosphoglycerol kinase (PGK) promoter, and the EFla promoter. Inducible promoters are regulated by exogenously supplied compounds, including, the zinc-inducible sheep metallothionine (MT) promoter, the dexamethasone (Dex)-inducible mouse mammary tumor virus (MMTV) promoter, the T7 polymerase promoter system; the ecdysone insect promoter, the tetracycline-repressible system, the tetracycline-inducible system, the RU486-inducible system and the rapamycin-inducible system. Other types of inducible promoters which may be useful in this invention are those which are regulated by a specific physiological state, e.g., temperature, acute phase, a particular differentiation state of the cell, or in replicating cells only.

In another embodiment, the native promoter for the genomic sequence encoding miRNA miR-1298, miR-200a-3p, miR-34a-5p, miR-375-3p, miR-509-5p, miR-879-5p, miR-466i-5p, miR-434-5p or encoding pre-miRNA mir-1298, mir-200a, mir-34a, mir-375, mir-509, mir-879, mir-466i, or mir-434 will be used. The native promoter may be preferred when it is desired that expression of said genomic sequence encoding said miRNA or said pre-miRNA should mimic the native expression. The native promoter may be used when expression of said genomic sequence encoding said miRNA or said pre-miRNA must be regulated temporally or developmentally, or in a tissue-specific manner, or in response to specific transcriptional stimuli. In a further embodiment, other native expression control elements, such as enhancer elements, polyadenylation sites or Kozak consensus sequences may also be used to mimic the native expression. In another embodiment, the genomic sequence encoding said miRNA, said pre-miRNA or other desirable product to be expressed is operably linked to a tissue-specific promoter. For instance, if expression in skeletal muscle is desired, a promoter active in muscle should be used. These include the promoters from genes encoding skeletal α-actin, myosin light chain 2A, dystrophin, muscle creatine kinase, as well as synthetic muscle promoters with activities higher than naturally-occurring promoters. Examples of promoters that are tissue-specific are known for liver [albumin, Miyatake et al. J Virol, 71:5124-32 (1997); hepatitis B virus core promoter, Sandig et al, Gene Ther., 3:1002-9 (1996); and alpha-fetoprotein (AFP), Arbuthnot et al, Hum. Gene Ther, 7:1503-14 (1996)], bone [osteocalcin, Stein et al, Mol. Biol. Rep., 24:185-96 (1997); and bone sialoprotein, Chen et al, J Bone Miner. Res., 11:654-64 (1996)], lymphocytes [CD2, Hansal et al., J Immunol, 161:1063-8 (1998); immunoglobulin heavy chain; T cell receptor a chain], neuronal [neuron-specific enolase (NSE) promoter, Andersen et al. Cell. Mol. Neurobiol, 13:503-15 (1993); neurofilament light-chain gene, Piccioli et al., 1991, Proc. Natl. Acad. Sci. USA, 88:5611-5 (1991); the neuron-specific vgf gene, Piccioli et al, Neuron 15:373-84 (1995)], lung [surfactant protein A, Bruno et al, J Biol Chem, 270, 6531-6536 (1996); surfactant protein B, Margana and Boggaram, J Biol Chem, 272, 3083-3090 (1997); surfactant protein C, Glasser et al, Am J Physiol, 261, L349-356 (1991)], skin [keratin K14, Staggers et al, Gene, 153, 297-298 (1995)], kidney [Na⁺/glucose cotransporter gene, Kanai et al, J Clin Invest, 93(1), 397-404 (1994); Ksp-cadherin gene, Igarashi et al, Am J Physiol, 277(4 Pt 2), F599-610 (1999); Na⁺/K⁺/Cl cotransporter gene, Igarashi et al, J Biol Chem, 271(16):9666-74 (1996); Podocin, Buchholz et al, Nucleic Acids Res, 24(21):4256-62 (1996); γ-glutamyl transpeptidase, Indra et al, Nucleic Acids Res, 27(22), 4324-7 (1999)]; among others.

Preferably, a promoter referred to herein can be derived from the cytomegalovirus (CMV) minimal promoter and, more preferably, from human CMV (hCMV) such as the hCMV immediate early promoter derived minimal promoter as described in, e.g., Gossen and Bujard (Proc. Natl. Acad. Sci. USA, 1992, 89: 5547-5551). Modified promoters also may be used, including insertion and deletion mutation of native promoters and combinations or permutations thereof.

Alternatively, the polynucleotide encoding miRNA miR-1298, miR-200a-3p, miR-34a-5p, miR-375-3p, miR-509-5p, miR-879-5p, miR-466i-5p, miR-434-5p or the polynucleotide encoding pre-miRNA mir-1298, mir-200a, mir-34a, mir-375, mir-509, mir-879, mir-466i, or mir-434 for use according to the invention may further comprise a polynucleotide of interest under operative control of the transcriptional regulatory region. The skilled person will appreciate that the polynucleotides of interest for use in the polynucleotides according to the present invention will depend on the intended use of the vector. Particularly preferred polynucleotides include reporter genes, when the polynucleotides of the invention are used as research tools or for imaging purposes, polynuclotides encoding polypeptides when the polynucleotides are used for gene therapy in diseases characterised by a lack of function of said polypeptide and polynucleotides which are capable of silencing the expression of target genes, adequate in those cases wherein the polynucleotides according to the invention are used for the treatment of diseases associated with an excessive expression of a given gene, either endogenous to the organism being treated or heterologous.

In a particular embodiment, the polynucleotide encoding miRNA miR-1298, miR-200a-3p, miR-34a-5p, miR-375-3p, miR-509-5p, miR-879-5p, miR-466i-5p, miR-434-5p, or the polynucleotide encoding pre-miRNA mir-1298, mir-200a, mir-34a, mir-375, mir-509, mir-879, mir-466i or mir-434 for use according to the invention, as previously described, is provided within a vector.

Preferably, the vector comprising the polynucleotide encoding miRNA miR-1298, miR-200a-3p, miR-34a-5p, miR-375-3p, miR-509-5p, miR-879-5p, miR-466i-5p, miR-434-5p, or the polynucleotide encoding pre-miRNA mir-1298, mir-200a, mir-34a, mir-375, mir-509, mir-879, mir-466i or mir-434 for use according to the present invention further comprises selectable markers for propagation and/or selection in a host.

A vector may be characterized by one or a small number of restriction endonuclease sites at which such DNA sequences may be cut in a determinable fashion without the loss of an essential biological function of the vector, and into which a DNA fragment may be spliced in order to bring about its replication and cloning. A vector may further contain a marker suitable for use in the identification of cells transformed with the vector.

The vector for use according to the present invention usually comprises regions adequate for inserting a polynucleotide of interest (e.g. the polynucleotide encoding miRNA or pre-miRNA of the invention), so that said polynucleotide is under operative control of the transcriptional regulatory region. In a preferred embodiment, the polynucleotide encoding miRNA miR-1298, miR-200a-3p, miR-34a-5p, miR-375-3p, miR-509-5p, miR-879-5p, miR-466i-5p, or miR-434-5p for use according to the invention comprises a polylinker downstream of the transcriptional regulatory region. The terms "polylinker" and "multiple cloning site" are used herein interchangeably to refer to refer to a region of DNA that contains one or more commonly used restriction sites allowing the insertion of a DNA fragment (e.g., gene of interest) into the expression construct.

One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e. g., bacterial vectors having a bacterial origin of replication and episomal mammalian vectors). Other vectors (e. g., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (e. g., replication defective retroviruses, adenoviruses and adeno-associated viruses), which serve equivalent functions.

In a preferred embodiment of the vector of the present invention, said vector is an expression vector. More preferably, in the vector of the invention, the transcription control sequence is operatively linked to a nucleic acid sequence to be expressed. Such operative linkage, preferably, allows expression of the said nucleic acid sequence in eukaryotic cells or isolated fractions thereof. In principle, regulatory elements ensuring expression in eukaryotic cells, preferably mammalian cells, are well known in the art. They, preferably, comprise regulatory sequences ensuring initiation of transcription as comprised by the transcription control sequence of the present invention as well as poly-A signals ensuring termination of transcription and stabilization of the transcript. Additional regulatory elements may be included into the vector such as transcriptional as well as translational enhancers. In this context, suitable expression vectors are known in the art such as vectors derived from retroviruses including lentiviruses, adenovirus, cytomegalovirus, adeno-associated viruses, measles virus, vaccinia virus, herpes viruses, or bovine papilloma virus, may be used for delivery of the vector of the invention into targeted cell population. Methods which are well known to those skilled in the art can be used to construct recombinant viral vectors.

Among the different vectors that may be used as all-in-one vectors in the context of the invention, lentiviral vectors are preferred because they are one of the most promising vectors for gene transfer in primary human cells, they are highly efficient and do not express any viral gene that could alter normal cellular physiology.

As used herein, the term "lentivirus" refers to a group (or scientific genus) of retroviruses that in nature give rise to slowly developing disease due to their ability to incorporate into a host genome. Modified lentiviral genomes are useful as viral vectors for the delivery of a nucleic acid sequence to a cell. An advantage of lentiviruses for infection of cells is the ability for sustained transgene expression. These viruses include in particular Human Immunodeficiency Virus type 1 (HIV-1), Human Immunodeficiency Virus type 2 (HIV-2), Simian Immunodeficiency Virus (SIV), Feline Immunodeficiency Virus (FIV), Equine Infectious Anaemia Virus (EIAV), Bovine Immunodeficiency Virus (BIV), Visna Virus of sheep (VISNA) and Caprine Arthritis-Encephalitis Virus (CAEV). Lentiviral vectors are well known in the art (see, for example, U.S. Pat. Nos. 6,013,516 and 5,994,136).

Lentiviral vectors are well known in the art (see, for example, U.S. Pat. Nos. 6,013,516 and 5,994,136). Recombinant lentiviral vectors are capable of infecting non-dividing cells and can be used for both *in vivo* and *ex vivo* gene transfer and expression of nucleic acid sequences. For example, recombinant lentiviruses capable of infecting a non-dividing cell wherein a suitable host cell is transfected with two or more vectors carrying the packaging functions, namely *gag, pol* and *env,* as well as *rev* and *tat* is described in U.S. Pat. No. 5,994,136. One may target the recombinant virus by linkage of the envelope protein with an antibody or a particular ligand for targeting to a receptor of a particular cell-type. By inserting a sequence (including a regulatory region) of interest into the viral vector, particularly a polynucleotide encoding miRNA miR-1298, miR-200a-3p, miR-34a-5p, miR-375-3p, miR-509-5p, miR-879-5p, miR-466i-5p, miR-434-5p, or a polynucleotide encoding pre-miRNA mir-1298, mir-200a, mir-34a, mir-375, mir-509, or mir-879, mir-466i, mir-434 along with another gene which encodes the ligand for a receptor on a specific target cell, for example, the vector is now target-specific. The recombinant lentiviruses according to the invention may be genetically modified in such a way that certain genes constituting the native infectious virus are eliminated and replaced with a nucleic acid sequence of interest to be introduced into the target cells.

The lentiviral vector can integrate into the genome of the host cell. The genetic material thus transferred is then transcribed and possibly translated into proteins inside the host cell. When the lentiviral vector is a non-integrative lentiviral vector, the vector is present in episomal forms. The lentiviral vector according to the present invention, in addition to the polynucleotide encoding miRNA miR-1298, miR-200a-3p, miR-34a-5p, miR-375-3p, miR-509-5p, miR-466i-5p, miR-434-5p or miR-879-5, the polynycleotide encoding pre-miRNA mir-1298, mir-200a, mir-34a, mir-375, mir-509, or mir-879, mir-466i, or mir-434 and/or the transcriptional regulatory sequence as described above, may further comprise additional elements which help to improve expression of the nucleotide sequence, particularly miRNA sequence, encoded within the vector. Additional elements include:
- Regions required for the integration of the vector into the genome of the target cell such as the Long-terminal repeats (LTRs) which flank the all-in-one vector defined herein. Thus, a lentiviral vector for use according to the invention preferably comprises a 5' LTR and a 3' LTR. "5' LTR" refers to a 5' retroviral or lentiviral long terminal repeat, which may or may not be modified from its corresponding native 5' LTR by deleting and/or mutating endogenous sequences and/or adding heterologous sequences. The 5' LTR may be natural or synthetic. "3' LTR" refers to a 3' retroviral or lentiviral long terminal repeat, which may or may not be modified from its corresponding native (i.e., that existing in the wild-type retrovirus) 3' LTR by deleting and/or mutating endogenous sequences and/or adding heterologous sequences. The 3' LTR may be natural or synthetic.
- An encapsidation sequence such as the lentiviral Psi (ψ) sequence,
- Sequences enhancing the RNA nuclear export, advantageous during the production, such as the sequence comprising the HIV-1 REV response element (RRE) sequence. Another sequence, usable in the context of the present invention, which enhances the RNA nuclear export, is the CTE sequence (Oh et al, 2007, Retrovirology. 2007 Jun 5;4:38.). These sequences are also useful for determining the copy number of the integrated lentiviral vectors.
- Sequences enhancing the nuclear import of the retrotranscribed viral DNA, such as the the lentiviral cPPT CTS (flap) sequence from, without limitation, HIV-1, HIV-2, SIV, FIV, EIAV, BIV, VISNA and CAEV.
- Posttranscriptional regulation elements may be selected from Woodchuck hepatitis virus responsive element (WPRE), APP UTR5' region and TAU UTR3'.
- One or more insulator sequences selected from the group consisting of, for example, MAR, SAR, S/MAR, scs and scs' sequences.
- Additional transcriptional regulatory elements: These are regions which help to improve expression of the genes encoded within the vector. In particular, the vector may incorporate the wood-chuck hepatitis virus post-transcriptional regulatory element (WPRE) at the 3' untranslated region (Paterna et al., 2000, Gene Ther. 7: 1304-1311).

In another embodiment, the lentiviral vector is another form of self-inactivating (SIN) vector as a result of a deletion in the 3' long terminal repeat region (LTR). Preferably, the vector contains a deletion within the viral promoter. The LTR of lentiviruses such as the HIV LTR contains a viral promoter. Although this promoter is relatively inefficient, when transactivated by e.g. tat, the promoter is efficient because tat-mediated transactivation increases the rate of transcription about 100 fold. However, the presence of the viral promoter can interfere with heterologous promoters operably linked to a transgene. To minimize such interference and better regulate the expression of transgenes, the lentiviral promoter may be deleted.

miRNA expression vectors are commercially allowable and include, without limitation, miRNA expression vectors pmR-ZsGreen1 (catalog No. 632541) and pmR-mCherry Vector (catalog No. 632542) from Clontech, pMIR-REPORT™ miRNA Expression Reporter Vector System (catalog No. AM5795) from Life Technologies, HIV-based or FIV-based lentivectors from System Biosciences, and miRNA mammalian expression vectors (catalog Nos. MIR-EXP-GP-C and MIR-EXP-C) from Cell Biolabs. Lentiviral vectors carrying miRNA libraries such as the pLemiR plasmid have been described (Open Biosystems) wherein the sequence encoding the miRNA is under the control of a CMV promoter. Since these plasmids encode for the precursor form of the miRNA (the pri-miRNA), activation by the cellular machinery is required before the miRNA can exert its biological effects.

Lentiviral expression vectors are also commercially available including, without limitation, the human pre-microRNA Expression Construct Lenti-miR-1298 (Catalog No. PMIRH1298PA-1) from System Biosciences.

The administration of the agent for use of the invention can be performed by different routes, for instance, enteral, inhalation, intravenous, intraperitoneal, subcutaneous, intramuscular, intratracheal, sublingual, topical, intradermal, subcutaneous, intranasal, transrectal, transvaginal, transdermic or intrabronchial, and it can be administered locally or systemically or directly to the target site. A review of the different administration routes of active substances, of the excipients to be used and the manufacturing procedures can be found in Tratado de Farmacia Galénica, C. Faulli i Trillo, Luzán 5, S.A. de Ediciones, 1993 and in Remington's Pharmaceutical Sciences (A.R. Gennaro, Ed.), 20th edition, Williams & Wilkins PA, USA (2000). The agent for use of the invention can occur at any pharmaceutical form of administration considered appropriate for the selected administration route, for example, by systemic, oral, parenteral or topical administration, for which it will include the pharmaceutically acceptable excipients necessary for formulation of the desired method of administration.

The dosage regimen will be established by the physician and the clinical factors. As it is well known in medicine, the dosages depend on many factors, including the physical characteristics of the patient (age, size, sex), the administration route used, the severity of the disease, the particular compound used and the pharmacokinetic properties of the subject.

The effective quantity of the compounds of the invention can vary within a wide range and, in general, will vary depending on the particular circumstances of application, duration of the exposure and other considerations.

Solid dosage forms for oral administration may include conventional capsules, sustained release capsules, conventional tablets, sustained-release tablets, chewable tablets, sublingual tablets, effervescent tablets, pills, suspensions, powders, granules and gels. At these solid dosage forms, the active compounds can be mixed with at least one inert excipient such as sucrose, lactose or starch. Such dosage forms can also comprise, as in normal practice, additional substances other than inert diluents, e.g. lubricating agents such as magnesium stearate. In the case of capsules, tablets, effervescent tablets and pills, the dosage forms may also comprise buffering agents. Tablets and pills can be prepared with enteric coatings.

Liquid dosage forms for oral administration may include emulsions, solutions, suspensions, syrups and elixirs pharmaceutically acceptable containing inert diluents commonly used in the technique, such as water. Those compositions may also comprise adjuvants such as wetting agents, emulsifying and suspending agents, and sweetening agents, flavoring and perfuming agents.

Injectable preparations, for example, aqueous or oleaginous suspensions, sterile injectable may be formulated according with the technique known using suitable dispersing agents, wetting agents and/or suspending agents. Among the acceptable vehicles and solvents that can be used are water, Ringer's solution and isotonic sodium chloride solution. Sterile oils are also conventionally used as solvents or suspending media.

For topical administration, compounds of the invention can be formulated as creams, gels, lotions, liquids, pomades, spray solutions, dispersions, solid bars, emulsions, microemulsions and similars which may be formulated according to conventional methods that use suitable excipients, such as, for example, emulsifiers, surfactants, thickening agents, coloring agents and combinations of two or more thereof.

Additionally, the compounds of the invention may be administered in the form of transdermal patches or iontophoresis devices. In one embodiment, the compounds of the invention are administered as a transdermal patch, for example, in the form of sustained-release transdermal patch. Suitable transdermal patches are described in more detail in, for example, US5262165, US5948433, US6010715 and US6071531.

Compositions comprising the compounds of the invention can additionally include conventional excipients, i.e. pharmaceutically acceptable carriers suitable for parenteral application which do not react damaging with the active compounds. Suitable pharmaceutically acceptable vehicles include, for example, water, salt solutions, alcohol, vegetable oils, polyethylene glycols, gelatin, lactose, amylose, magnesium stearate, talc, surfactants, silicic acid, viscous paraffin, perfume oil, monoglycerides and diglycerides of fatty acids, fatty acid esters petroetrals, hydroxymethyl cellulose, polyvinylpyrrolidone and similars.

Several drug delivery systems are known and can be used to administer the compounds or compositions of the invention, including, for example, encapsulation in liposomes, microbubbles, emulsions, microparticles, microcapsules and similars. The required dosage can be administered as a single unit or in a sustained release form.

Sustainable-release forms and appropriate materials and methods for their preparation are described in, for example, "Modified-Release Drug Delivery Technology", Rathbone, M. J. Hadgraft, J. and Roberts, M. S. (eds.), Marcel Dekker, Inc., New York (2002), "Handbook of Pharmaceutical Controlled Release Technology", Wise, D. L. (ed.), Marcel Dekker, Inc. New York, (2000). In one embodiment of the invention, the orally administrable form of a compound according to the invention is in a sustained release form further comprises at least one coating or matrix. The coating or sustained release matrix include, without limitation, natural polymers, semisynthetic or synthetic water-insoluble, modified, waxes, fats, fatty alcohols, fatty acids, natural semisynthetic or synthetic plasticizers, or a combination of two or more of them.

Enteric coatings may be applied using conventional processes known to experts in the art, as described in, for example, Johnson, J. L., "Pharmaceutical tablet coating", Coatings Technology Handbook (Second Edition), Satas, D. and Tracton, A. A. (eds), Marcel Dekker, Inc. New York, (2001), Carstensen, T., "Coating Tablets in Advanced Pharmaceutical Solids", Swarbrick, J. (ed.), Marcel Dekker, Inc. New York (2001), 455-468.

Even though individual needs vary, determination of optimal ranges for effective amounts of the compound of the invention belongs to the common experience of those experts in the art. In general, the dosage needed to provide an effective amount of such compound, which can be adjusted by one expert in the art will vary depending on age, health, fitness, sex, diet, weight, degree of alteration of the receptor, frequency of treatment and the nature and extent of impairment or illness, medical condition of the patient, route of administration, pharmacological considerations such as activity, efficacy, pharmacokinetic and toxicology profile of the particular compound used, if using a system drug delivery, and if the compound is administered as part of a combination of drugs.

The amount of the compound according to the invention that is effective in the treatment of a particular disorder or condition will depend on the nature of the disorder or condition, and can be determined by conventional clinical techniques, including reference to Goodman and Gilman, supra; The Physician's Desk Reference, Medical Economics Company, Inc., Oradell, NJ, 1995, and Drug Facts and Comparisons, Inc., St. Louis, MO, 1993. The precise dose to use in the formulation will also depend on the route of administration, and severity of the disease or disorder, and should be decided in the physician's opinion and the patient's circumstances.

In the particular case that the agent for use according to the invention is a miRNA or its corresponding miRNA, as previously described, it is formulated in a composition intended for use in gene therapy. In the particular case that the agent for use according to the invention is selected from the group consisting of a polynucleotide encoding a miRNA or a polynucleotide encoding a pre-miRNA, by way of illustration and not limitation, it may be formulated in a pharmaceutical composition containing a viral or non-viral vector. By way of illustration and not limitation, said vectors, may be viral, for example, based on retrovirus, adenovirus, etc., or nonviral such as ADN-liposome, ADN-polymer, ADN-polymer-liposome complexes, etc. [see "Nonviral Vectors for Gene Therapy", edited by Huang, Hung and Wagner, Academic Press (1999)]. Said vectors, which contain a polynucleotide according to the invention, may be administered directly to humans or animals by conventional methods. Alternatively, said vectors may be used to transform, or transfect or infect cells, for example, mammal cells, including human, *ex vivo,* which subsequently will be implanted into a human body or an animal to obtain the desired therapeutic effect. For administration to a human body or an animal, said cells will be formulated in a suitable medium that will have no adverse influence on cell viability.

The invention is described in detail below by means of the following examples which are to be construed as merely illustrative and not limitative of the scope of the invention.

### EXAMPLE 1. Identification of biomarkers of diffuse alveolar damage (DAD)

### Animal model

Sprague-Dawley rats weighing between 325 and 375 g were randomized into two groups:
(a) ventilated with low tidal volume (control, n = 10): tidal volume (TV) = 9 ml/kg, positive end-expiratory pressure (PEEP, or the pressure at the end of the exhalation) = 5 cm H₂0 for 2.5 h; and
(b) ventilated with high tidal volume (n = 19): tidal volume = 25 ml/kg, PEEP = 0 for 2.5 h.

After the period of ventilation, the animals were sacrificed and the lungs were removed. The left lung was included in formalin for histological analysis. The right and left lung were immediately included in liquid nitrogen and frozen at -80 °C up to microRNA extraction according to conventional techniques.

As a result, the histopathological analysis showed that in the group of animal receiving ventilation with high tidal volumen, 11 of the 19 animals showed DAD. None of the animals who receive low tidal volume showed DAD.

Accordingly, based on the ventilation strategy and histopathology results, animals were classified into 3 groups:
a) rats ventilated with low tidal volume (n = 10)
b) rats ventilated with high tidal volume that do not develop DAD (n = 8)
c) rats ventilated with high tidal volume that develop DAD (n = 11)

### Massive microRNA sequencing using RNA-seq

Sequencing was initiated by conducting small-RNA libraries using the kit NEBNext ® Small RNA Library Prep Set for Illumina® (Multiplex Compatible) (New England Biolabs) starting with 2 ug of total RNA. A quality control was performed with High Sensitivity DNA chip kit (Agilent, USA) in Bioanalyzer (Agilent, USA). The libraries were quantified with the Qubit dsDNA broad range kit (Invitrogen, USA). 10 indexed libraries per lane were clustered at a final concentration of 1 pM. A single read sequencing of 72 cycles was conducted on a Illumina GAIIx with the kit TruSeq SBS Kit v5-GA (Illumina, USA), following the instructions of the manufacturer.

### Identification of differentially expressed microRNA

The FASTQ files generated by the sequencer were analyzed using the Casava v1.8.2 package (Illumina Inc ®) in order to generate separate readings and according to the index corresponding to each sample. Then the programs cutadapt (http://code.google.com/p/cutadapt/), FASTX-Toolkit (http://hannonlab.cshl.edu/fastx_toolkit/) and programs developed in Python language were used to remove the 3 'sequences and to filter by quality. Sequences consisting of 17 to 26 nucleotides and appearing at least in duplicate were selected.

In order to identify microRNA, all selected sequences were introduced in miRanalyzer (http://bioinfo5.ugr.es/miRanalyzer dev/miRanalyzer.php) which uses as a database repository to miRBase version 19 (http://www.mirbase.org)

microRNA annotation was made from known genome sequences of *Rattus norvegicus* (mature and pre-microRNAs) allowing one difference ("mismatch"). The sequences that were not found in the genome of *Rattus norvegicus* were aligned against the genomes from other mammals (without allowing any difference) based on databases transcripts (Rfam, RepBase, piwi RNA and tRNA), one mismatch being allowed, and finally against *Rattus norvegicus* genome rn4 (one mismatch being allowed).

microRNA profiles for each sample were constructed from these outputs of miRanalyzer, using and counting only the sequences that were annotated as rat microRNA or homologous to any of the mammalian microRNAs (considering the species of miRBase database, including *Mus musculus* and *Homo sapiens*).

As a result of the counts for the 29 samples, 559 mature microRNA (472 annotated in rat and 87 as homologous to other mammalian microRNAs) were identified. These counts were analyzed with edgeR for differential expression data. By requiring that normalized counts per million mapped sequences (cpm) counts were at least 1 in at least 8 samples, the number of relevant microRNA was reduced to 390. Normalization and estimation of common dispersion was performed using default parameters. To estimate the tagwise dispersion, the prior.df parameter was changed to 6.

Mature miRNA with differential expression between different conditions (low tidal volume, high tidal volume without DAD and high tidal volume with DAD) were identified using these values dispersion in the exact test implemented methods qCML in edgeR, for FDR < 5%.

Finally, 178 sequences were identified that were differentially expressed in any of the conditions (Table 2).

The following comparisons were established:
- low tidal volume vs. high tidal volume,
- low tidal volume vs. high tidal volume without DAD,
- low tidal volume vs. high tidal volume with DAD, and
- high tidal volume with DAD vs. high tidal volume without DAD.

The authors found that 17 miRNAs were significantly differentially expressed in at least 1 of the 4 comparisons as above. This list of miRNA is shown in Table 2 below.

**Table 2. List of miRNAs differentially expressed among the three groups of animals analyzed.**

| | Low vs. High tidal volume | | Low volume vs. High volume without DAD | | Low volume vs. High volume with DAD | | High volume without vs. with DAD | |
|---|---|---|---|---|---|---|---|---|
| | logFC | p value | logFC | p value | logFC | p value | logFC | p value |
| **bta-miR-2478** | 0.529 | 0.764 | -0.141 | 0.966 | 0.874 | 0.035 | 1.015 | 0.0266 |
| **hsa-miR-486-5p** | -0.010 | 1.000 | -0.494 | 0.498 | 0,263 | 0.927 | 0.757 | 0.049 |
| **mmu-miR-466i-5p** | -2.055 | 0.229 | -5.069 | 0.018 | -1.3247 | 0.657 | 3.743 | 0.178 |
| **rno-miR-125b-1-3p** | 0.562 | 0.133 | 0.206 | 0.887 | 0.790 | 0,001 | 0.584 | 0.051 |
| **rno-miR-1298** | -0.465 | 0.876 | -0.200 | 1.000 | -0.910 | 0.048 | -0.891 | 0.071 |
| **rno-miR-132-5p** | 2.516 | 0.051 | 0.540 | 0.926 | 3.129 | <0.001 | 2.593 | 0.013 |
| **rno-miR-133a-3p** | 1.183 | 0.051 | 0.843 | 0.496 | 1.388 | 0.012 | 0.545 | 0.523 |
| **rno-miR-200a-3p** | -0.064 | 1.000 | 0.166 | 0.837 | -0.257 | 0.458 | -0.422 | 0.049 |
| **rno-miR-208a-3p** | 3.298 | 0.072 | 2.007 | 0.540 | 3.799 | 0.022 | 1.731 | 0.453 |
| **rno-miR-21-3p** | 1.325 | 5.6E-07 | 1.226 | <0.001 | 1.394 | 1.7E-06 | 0.168 | 0.826 |
| **rno-miR-27a-5p** | 1.911 | 1.5E-14 | 1.270 | 1.5E-08 | 2.242 | 4.6E-31 | 0.972 | <0.001 |
| **rno-miR-34a-5p** | -0.066 | 1.000 | 0.266 | 0.687 | -0.367 | 0.246 | -0.633 | 0.005 |
| **rno-miR-375-3p** | -0.127 | 1.000 | 0.142 | 0.887 | -0.361 | 0.247 | -0.503 | 0.049 |
| **rno-miR-434-5p** | 0.957 | 0.157 | 1.361 | 0.017 | 0.568 | 0.687 | -0.793 | 0.209 |
| **rno-miR-490-3p** | 0.804 | 0.062 | 0.586 | 0.498 | 0.944 | 0.021 | 0.359 | 0.577 |
| **rno-miR-509-5p** | 0.518 | 1.000 | 1.225 | 0.311 | -0.500 | 0.947 | -1.726 | 0.049 |
| **rno-miR-879-5p** | -0.077 | 1.000 | 0.246 | 0.755 | -0.386 | 0.361 | -0.631 | 0.027 |

### Selection of the most informative miRNA

Based on the previous data, the authors performed a data mining analysis in order to prioritize those miRNA with a diagnostic marker value for DAD.

The data mining program Weka (http://www.cs.waikato.ac.nz/ml/weka/) was used to identify those miRNA that best classify the three conditions under analysis (low tidal volume, high tidal volume without DAD and high tidal volume with DAD).

A number of classifying systems were tested (see Table 3), the J48 algorithm being the most accurate and parsimonic.

**Table 3. Data mining data based on the 17 miRNAs differentially expressed identified by the inventors**

| Classifier | Test option | Correctly Classified | group | TP rate | FP rate | Precission | Recall | F-Measure | ROC curve |
|---|---|---|---|---|---|---|---|---|---|
| **J48** | all samples | 97% | LV | 1 | 0 | 1 | 1 | 1 | 1 |
| | | | HV, DAD (+) | 1 | 0.056 | 0.917 | 1 | 0.957 | 0.972 |
| | | | HV, DAD (-) | 0.875 | 0 | 1 | 0.875 | 0.933 | 0.967 |
| **J48** | cross-validation (folds 5) | 86% | LV | 1 | 0.053 | 0.909 | 1 | 0.952 | 0.974 |
| | | | HV, DAD (+) | 0.909 | 0.167 | 0.769 | 0.909 | 0.833 | 0.866 |
| | | | HV, DAD (-) | 0.625 | 0 | 1 | 0.625 | 0.769 | 0.878 |
| **IBK** | all samples | 100% | LV | 1 | 0 | 1 | 1 | 1 | 1 |
| | | | HV, DAD (+) | 1 | 0 | 1 | 1 | 1 | 1 |
| | | | HV, DAD (-) | 1 | 0 | 1 | 1 | 1 | 1 |
| | cross-validation (folds 5) | | LV | 0.900 | 0.053 | 0.900 | 0.900 | 0.900 | 0.887 |
| | | | HV, DAD (+) | 0.818 | 0.111 | 0.818 | 0.818 | 0.818 | 0.851 |
| | | | HV, DAD (-) | 0.875 | 0.048 | 0.875 | 0.875 | 0.875 | 0.925 |
| **PART** | all samples | 97% | LV | 1 | 0 | 1 | 1 | 1 | 1 |
| | | | HV, DAD (+) | 1 | 0.056 | 0.917 | 1 | 0.957 | 0.972 |
| | | | HV, DAD (-) | 0.875 | 0 | 1 | 0.875 | 0.933 | 0.967 |
| | cross-validation (folds 5) | 83% | LV | 0.900 | 0.053 | 0.900 | 0.900 | 0.900 | 0.924 |
| | | | HV, DAD (+) | 0.909 | 0.167 | 0.769 | 0.909 | 0.833 | 0.860 |
| | | | HV, DAD (-) | 0.625 | 0.048 | 0.833 | 0.625 | 0.714 | 0.842 |
| **Random Tree** | all samples | 100% | LV | 1 | 0 | 1 | 1 | 1 | 1 |
| | | | HV, DAD (+) | 1 | 0 | 1 | 1 | 1 | 1 |
| | | | HV, DAD (-) | 1 | 0 | 1 | 1 | 1 | 1 |
| | cross-validatio (folds 5) | 66% | LV | 0.400 | 0.105 | 0.667 | 0.400 | 0.500 | 0.647 |
| | | | HV, DAD (+) | 0.909 | 0.222 | 0.714 | 0.909 | 0.800 | 0.843 |
| | | | HV, DAD (-) | 0.625 | 0.190 | 0.556 | 0.625 | 0.588 | 0.717 |

| | | | | | | | | | |
|---|---|---|---|---|---|---|---|---|---|
| LV: low tidal volume; HV: high tidal volume | | | | | | | | | |

From the differentially expressed miRNA identified above, miRNA-27a-5p was analyzed individually. Group classification (low tidal volume, high tidal volume without DAD and high tidal volume with DAD) based on miRNA-27a-5p is shown in Figure 1.

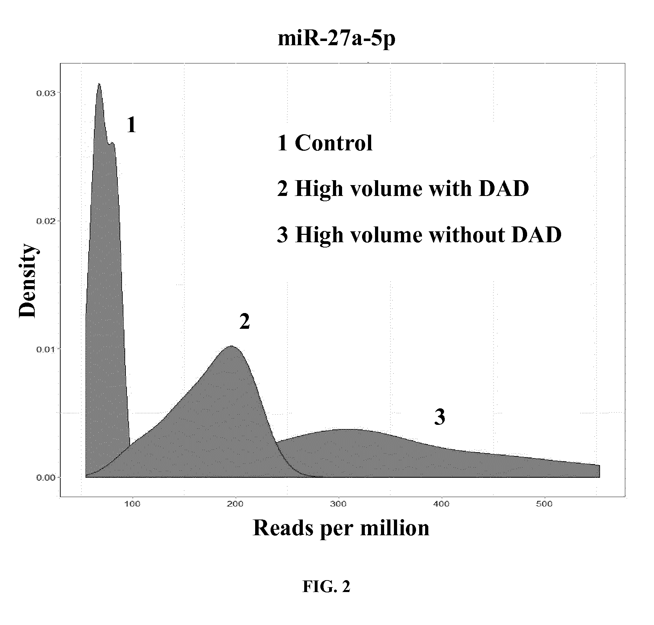

Figure 2 shows the distribution of mi-27a-5p expression levels in the three populations analyzed (low tidal volume or control, high tidal volume without DAD and high tidal volume with DAD), indicating that it was differentially expressed in the three groups of animals analyzed.

### miRNA expression analysis in rat serum samples

After miRNA selection based on the analysis of rat lung tissue samples, miRNA expression levels were then analyzed in rat serum samples.

Serum was obtained from the same rats in which the RNA-seq analysis was performed, and the expression of the two selected miRNA (miR-27a-5p and miR-486-5p) was measured by real-time PCR. miRNA was extracted from the rat serum samples according to standard protocols.

Expression data were normalized using the deltaCT method with a spike-in from *Caenorhabditis elegans.* The expression value of each microRNA was dichotomized according to the value of higher sensitivity and specificity in the ROC curve for the events ventilation with high tidal volume and DAD. The test was considered positive if greater that the cutting point and test was negative if equal or lower. Gene expression was measured in arbitrary units and is relative to the expression of the spike-in used.

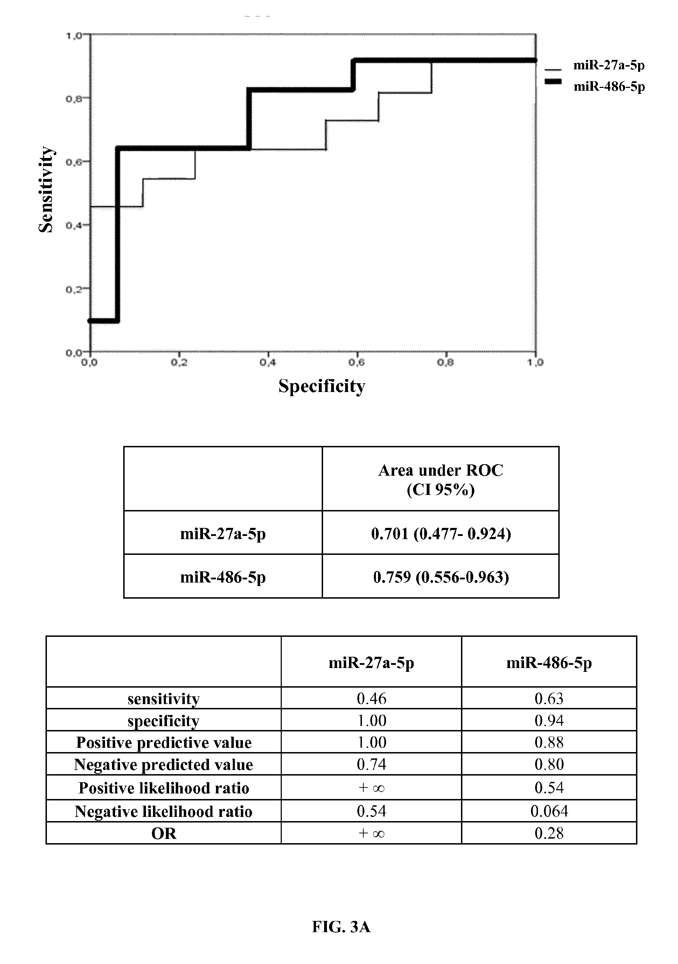

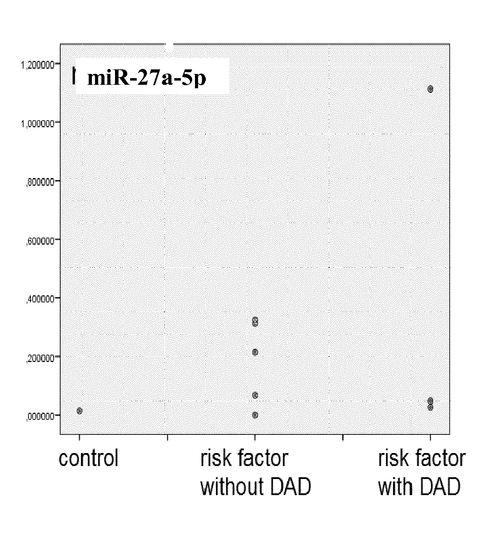

Selected miRNA miR-27a-5p and miR-486-5p, evaluated in serum samples, showed high specificity in DAD diagnosis. The discriminatory accuracy between DAD and non-DAD based on miR-27a-5p and miR-486-5p expression levels is shown in Figure 3A.

Sensitivity, specificity, positive and negative predictive value, and positive and negative likelihood ratio were calculated for both miRNAs (see Figure 3A).

### miRNA expression analysis in human lung samples

miR-27a-5p and miR-486-5p expression was analysed in lung samples (n=9) from subjects showing risk factors and suffering or not from DAD. Results are shown in Figure 3B.

### EXAMPLE 2. Qualification of miRNAs biomarkers in serum samples from septic patients at intensive care unit (ICU) admission

Next, the inventors analyzed the sensitivity, specificity and accuracy of miRNAs identified above in predicting mortality associated to mechanical ventilation. Additionally, the inventors evaluated the sensitivity, specificity and accuracy of miRNAs in diagnosing ARDS associated to patients having risk factors related to ARDS.

For the study, individuals aged more than 18 years at ICU of Getafe Universitary Hospital in Madrid, Spain, diagnosed of severe sepsis and requiring invasive mechanical ventilation were included. Subjects under second admission to the ICU, under immunosuppression treatment, with a life-expectancy lower than 24 hours or without informed consent were excluded from the study. The operative definition of severe sepsis and septic shock was that of ACCP/SCCM (American College of Chest Physicians/Society of Critical Care Medicine). The operative definition of ARDS was the Berlin definition.

Serum samples from those subjects selected for the study (n=66) were taken within the first 24 hours from ICU admission.

Gene expression was analyzed by real-time PCR, and expression values were normalized with a spike-in.

Univariate analysis results are shown in Table 4.

**Table 4. Parameters of the patients included in the study**

| | All (n=66) | Death (n=14) | Alive (n=52) | p | ARDS (n= 39) | Never ARDS (n= 27) | p |
|---|---|---|---|---|---|---|---|
| Age (years) | 70 | 68 | 70 | 0.71 | 70 | 65 | 0.49 |
| | (59-78) | (60-78) | (58-75) | | (61-78) | (58-75) | |
| Male | 51 (77.7) | 14 (85.71) | 39 (75.00) | 0.62 | 28 (71.8) | 23 (85.2) | 0.33 |
| SAPSII* | 48 (37-56) | 64(52-70) | 46(36-52) | <0.0 1 | 47 (39-55) | 50 (37-59) | 0.91 |
| SOFA* | 7(4-9) | 8(6.0-11.0) | 7(3-9) | 0.22 | 7 (5-10) | 7(4-9) | 0.48 |
| Length of ICU stay | 9 (5-19) | 8 (2-21) | 9 (6-16) | 0.30 | 1 (6-19) | 7 (5-19) | 0.19 |
| Lenght of hospital stay | 31(14-43) | 22(4-39) | 35 (16-55) | 0.04 | 31 (15-42) | 31 (12-56) | 0.96 |
| Days between hospital and ICU admission | 1 (0-5) | 2 (0-12) | 0(0-3) | 0.32 | 0 (0-5) | 1 (0-5) | 0.25 |

| Comorbidities | | | | | | | |
|---|---|---|---|---|---|---|---|
| Chronic alcohol intake | 8(12.1) | 2 (14.28) | 6(11.5) | 1.00 | 4(10.5) | 4(14.8) | 0.71 |
| Diabetes | 17 (25.8) | 2 (14.28) | 15(28.85) | 0.44 | 12 (30.8) | 5 (18.5) | 0.40 |
| Arterial hyperthension | 29 (43.9) | 9 (64.29) | 20 (38.46) | 0.15 | 20 (51.3) | 9 (33.3) | 0.23 |
| Heart failure (III or IV NYHA) | 6(9.1) | 2(14.28) | 4(7.69) | 0.60 | 4(10.5) | 2(7.4) | 0.71 |
| Coronary artherial disease | 5(7.6) | 2(14.28) | 3 (5.77) | 0.29 | 3(7.7) | 2(7.4) | 1.00 |
| Chronic liver disease | 6(9.1) | 1(7.14) | 5(9.62) | 1.00 | 2(5.1) | 6(14.8) | 0.22 |
| COPD | 16 (24.2) | 3 (21.43) | 13(25.00) | 1.00 | 9(23.1) | 7 (25.9) | 1.00 |

| Diagnosis at ICU admission | | | | | | | |
|---|---|---|---|---|---|---|---|
| Severe sepsis | 58 (87.9) | 12 (85.8) | 46 (88.5) | 0.796 | 36 (87.8) | 22 (88.0) | 0.20 |
| Haemorragic shock | 5 (7.6) | 1 (7.1) | 4(7.7) | | 4(9.6) | 1(4.0) | |
| Miscellaneuos | 3 (4.5) | 1 (7.1) | 2 (3.8) | | 1 (2.5) | 2 (8.0) | |
| Shock at ICU admission | 51(77.2) | 13 (92.85) | 38 (73.07) | 0.16 | 30(76.9) | 21 (77.7) | 1.00 |

| Clinical parameters | | | | | | | |
|---|---|---|---|---|---|---|---|
| Noradrenalina (mcg/kg/min) | 0.15 (0.0-0.46) | 0.29 (0.06-1.36) | 0.20 (1.50-5,24) | 0.15 | 0.18 (0.00 - 0.50) | 0.15 (0.00-0.42) | 0.88 |
| ratio Pa0₂/Fio0₂ | 213 (154-305) | 271(92-350) | 208 (166-297) | 0.94 | 200 (123-261) | 263 (187-336) | 0.03 |
| dynamic compliance (ml/cm H₂0) | 26 (20-36) | 23 (17-29) | 26 (21-37) | 0.29 | 29 (22-36) | 23 (17-34) | 0.15 |
| Arterial tension (mm Hg) | 110 (99-127) | 110(101-125) | 111 (96-127) | 0.64 | 110 (99-120) | 109 (98-128) | 0.76 |

| Analitical parameters | | | | | | | |
|---|---|---|---|---|---|---|---|
| miRNA-27a-5p (x10⁻⁵) | 6.74 (3.15-13.8) | 13.8 (6.61-39.3) | 5.44 (2.14-11.70) | <0.0 1 | 10.5(4.2-16.1) | 5.4 (1.8-10.2) | 0.02 |
| Procalcitonin | 7.9 (1.7-26.2) | 6.5(2.1-23.1) | 9.4 (1.7-26.2) | 0.77 | 7.9 (1.5-25.9) | 7.5 (1.7-26.4) | 0.75 |
| Lactate | 2.9 (1.6-5.2) | 3.1 (2.3-8.1) | 2.8 (1.5-5.2) | 0.26 | 2.9 (1.4-6.1) | 2.3 (1.6-4.9) | 0.40 |
| PCR | 155 (66-190) | 140(17-190) | 150(8-190) | 0.27 | 150 (60-190) | 135 (86-170) | 0.25 |
| Glucose (mg/dl) | 151 (107-199) | 131(191-159) | 158(115-199) | 0.22 | 147 (105-227) | 159 (115-182) | 0.80 |
| Creatinin (mg) | 1.44 (0.98-2.08) | 2.16 (1.37-2.76) | 1.38(0.94-2.00) | 0.09 | 1.43 (0.93-2.05) | 1.47 (1.16-2.13) | 0.71 |
| Hemoglobin (gr/dl) | 10.8 (9.6-13.1) | 10.9(9.9-14.0) | 10.7(9.6-12.7) | 0.44 | 10.7 (9.7-12.7) | 11.0 (9.6-13.2) | 0.68 |
| Leukocyte (x10³) | 10.0 (5.9-18.1) | 6.4(3.2-22.7) | 10.9 (6.3-15.7) | 0.83 | 11.3 (6.5-21.9) | 7.5 (4.2-15.3) | 0.04 |
| Platelet (x10³) | 173 (111-253) | 113 (107-720) | 202(120-254) | 0.12 3 | 200 (90 - 274) | 164 (111-245) | 0.84 |

| | | | | | | | |
|---|---|---|---|---|---|---|---|
| SAPS II: simplified acute physiology score II; SOFA: sequential organ failure assessment; ICU: intensive care unit; NYHA: New York heart association; COPD: chronic obstructive pulmonary disease | | | | | | | |

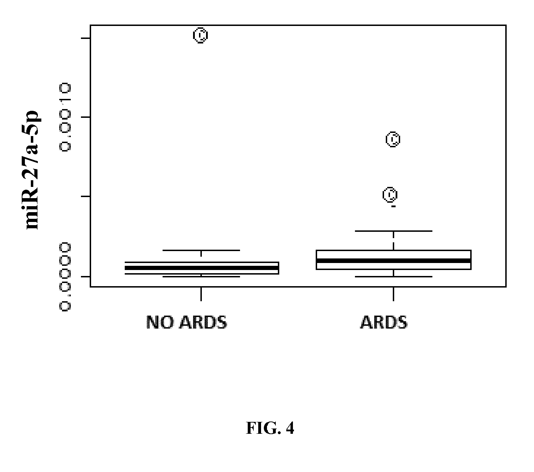

Figure 4 shows miR-27a-5p expression levels measured in serum samples for subjects with no ARDS or suffering from ARDS. miR-27a-5p expression levels were increased in those subjects suffering from ARDS.

A multivariate analysis of patient mortality was performed based on the following parameters:
- SAPSII (OR 1,05, CI95% 1.01-1.10, p=0.02)
- miR27a-5p (OR 1.06; CI95% 1.01-1.12; p=0.04)

Figure 5 shows the mortality ROC curve when miRNA-27a-5p expression level and/or SAPS II are analyzed, showing that the discriminative capacity is higher when the two parameters were analyzed in combination.

Figure 6 shows the mortality ROC curve when miRNA-27a-5p expression level or SAPS II were analyzed, in comparison with usual clinic biomarkers such as lactate and procalcitonine.

Table 5 shows miR-27a-5p expression levels in patients undergoing different degrees (no ARDS, mild, moderate, and severe) of ARDS.

**Table 5. miR-27a-5p expression levels determined in patients undergoing ARDS**

| ARDS severity | miR-27a-5p expression level p50 (p25-p75) |
|---|---|
| NoARDS | 5,4 (1,8 - 9,0) x10⁻⁵ |
| Mild ARDS | 6,8 (5,1 - 9,6) x10⁻⁵ |
| Moderate ARDS | 13,3 (5,2 - 17,1) x10⁻⁵ |
| Severe ARDS | 10,0 (1,5 -16,7) x10⁻⁵ |

## Claims

1. An *in vitro* method for the diagnosis of diffuse alveolar damage (DAD) in a subject that comprises
(i) determining the level of expression of at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof in a sample from the subject,
(ii) comparing the level of expression of said at least one miRNA to a reference value, and
(iii) correlating a variation in the expression level of said at least one miRNA respect to its reference value with the diagnosis of DAD.

2. An *in vitro* method according to the claim 1, wherein
- if the level of expression of at least one miRNA selected from the group consisting of miR-27a-5p, miR-486-5p, miR-2478, miR-125b-1-3p, miR-132-5p, miR-133a-3p, miR-208a-3p, miR-21-3p, miR-490-3p, and variants thereof is increased respect to its reference value, and/or
- if the level of expression of at least one miRNA selected from the group consisting of miR-1298, miR-200a-3p, miR-34a-5p, miR-375-3p, miR-509-5p, miR-879-5p, miR-466i-5p, miR-434-5p and variants thereof is decreased respect to its reference value,
then the subject is diagnosed with DAD.

3. The method according to any of claims 1 or 2, wherein the level of expression of miR-27a-5p and/or miR-486-5p is determined, and wherein if the level of expression of miR-27a-5p and/or miR-486-5p is increased respect to its reference value, then the subject is diagnosed with DAD.

4. An *in vitro* method for confirming a diagnosis of acute respiratory distress syndrome (ARDS) in a subject suspected to suffer from ARDS that comprises:
(i) determining the level of expression of at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof in a sample from the subject,
(ii) comparing the level of expression of said at least one miRNA to a reference value, and
(iii) correlating a variation in the expression level of said at least one miRNA respect to its reference value with the confirmation of the diagnosis of ARDS in said subject.

5. A method according to claim 5, wherein
- if the level of expression of at least one miRNA selected from the group consisting of miR-27a-5p, miR-486-5p, miR-2478, miR-125b-1-3p, miR-132-5p, miR-133a-3p, miR-208a-3p, miR-21-3p, miR-490-3p, and variants thereof is increased respect to its reference value, and/or
- if the level of expression of at least one miRNA selected from the group consisting of miR-1298, miR-200a-3p, miR-34a-5p, miR-375-3p, miR-509-5p, miR-879-5p, miR-466i-5p, miR-434-5p and variants thereof is decreased respect to its reference value,
then the diagnosis of ARDS is confirmed,
or, alternatively,
- if the level of expression of at least one miRNA selected from the group consisting of miR-27a-5p, miR-486-5p, miR-2478, miR-125b-1-3p, miR-132-5p, miR-133a-3p, miR-208a-3p, miR-21-3p, miR-490-3p, and variants thereof is decreased respect to its reference value, and/or
- if the level of expression of at least one miRNA selected from the group consisting of miR-1298, miR-200a-3p, miR-34a-5p, miR-375-3p, miR-509-5p, miR-879-5p, miR-466i-5p, miR-434-5p and variants thereof is increased respect to its reference value,
then the diagnosis of ARDS is not confirmed.

6. The method according to any of claims 4 or 5, wherein the level of expression of miR-27a-5p and/or miR-486-5p is determined, and wherein
if the level of expression of miR-27a-5p and/or miR-486-5p is increased respect to its reference value, then the diagnosis of ARDS is confirmed,
or, alternatively,
if the level of expression of miR-27a-5p and/or miR-486-5p is decreased respect to its reference value, then the diagnosis of ARDS is not confirmed.

7. An *in vitro* method for determining the prognosis of a subject undergoing mechanical ventilation that comprises
(i) determining the level of expression of at least one miRNA selected from the group consisting of miR-27a-5p, miR-486-5p, miR-2478, miR-466i-5p, miR-125b-1-3p, miR-1298, miR-132-5p, miR-133a-3p, miR-200a-3p, miR-208a-3p, miR-21-3p, miR-34a-5p, miR-375-3p, miR-434-5p, miR-490-3p, miR-509-5p, miR-879-5p, and variants thereof in a sample from the subject,
(ii) comparing the level of expression of said at least one miRNA to a reference value, and
(iii) correlating a variation in the expression level of said at least one miRNA respect to its reference value with the prognosis of said subject undergoing chemical ventilation.

8. Method according to claim 7, wherein
- if the level of expression of at least one miRNA selected from the group consisting of miR-27a-5p, miR-486-5p, miR-2478, miR-125b-1-3p, miR-132-5p, miR-133a-3p, miR-208a-3p, miR-21-3p, miR-490-3p, and variants thereof is increased respect to its reference value, and/or
- if the level of expression of at least one miRNA selected from the group consisting of miR-1298, miR-200a-3p, miR-34a-5p, miR-375-3p, miR-509-5p, miR-879-5p, miR-466i-5p, miR-434-5p and variants thereof is decreased respect to its reference value,
it is indicative of a bad prognosis of the subject undergoing mechanical ventilation,
or, alternatively, wherein
- if the level of expression of at least one miRNA selected from the group consisting of miR-27a-5p, miR-486-5p, miR-2478, miR-125b-1-3p, miR-132-5p, miR-133a-3p, miR-208a-3p, miR-21-3p, miR-490-3p, and variants thereof is decreased respect to its reference value, and/or
- if the level of expression of at least one miRNA selected from the group consisting of miR-1298, miR-200a-3p, miR-34a-5p, miR-375-3p, miR-509-5p, miR-879-5p, miR-466i-5p, miR-434-5p and variants thereof is increased respect to its reference value,
it is indicative of a good prognosis of the subject undergoing mechanical ventilation.

9. The method according to any of claims 7 or 8, wherein the level of expression of miR-27a-5p and/or miR-486-5p is determined, and wherein
if the level of expression of miR-27a-5p and/or miR-486-5p is increased respect to its reference value, it is indicative of a bad prognosis of the subject undergoing mechanical ventilation,
or, alternatively,
if the level of expression of miR-27a-5p and/or miR-486-5p is decreased respect to its reference value, it is indicative of a good prognosis of the subject undergoing mechanical ventilation.

10. Method according to any of claims 1 to 9, wherein the sample is selected from the group consisting of blood, serum, plasma, bronchoalveolar lavage, saliva, urine and a tissue sample.

11. Method according to claim 10, wherein the tissue sample is a lung tissue sample.

12. An agent for use in the prevention and/or treatment of DAD in a subject selected from:
- an agent inhibiting the expression of a miRNA selected from the group consisting of miR-27a-5p, miR-486-5p, miR-2478, miR-125b-1-3p, miR-132-5p, miR-133a-3p, miR-208a-3p, miR-21-3p and miR-490-3p, and
- an agent inducing the expression of a miRNA selected from the group consisting of miR-1298, miR-200a-3p, miR-34a-5p, miR-375-3p, miR-509-5p, miR-879-5p, miR-466i-5p and miR-434-5p.

13. An agent for use according to claim 12, wherein the agent inhibiting the expression of said miRNA is selected from the group consisting of an anti-miR and a tiny LNA.

14. An agent for use according to claim 12, wherein
- the agent inducing the expression of miR-1298 is selected from the group consisting of miR-1298, a miR-1298 variant, and agent inducing the expression of miR-1298 and an agent inducing the expression of a variant of miR-1298,
- the agent inducing the expression of miR-200a-3p is selected from the group consisting of miR-200a-3p, a miR-200a-3p variant, and agent inducing the expression of miR-200a-3p and an agent inducing the expression of a variant of miR-200a-3p,
- the agent inducing the expression of miR-34a-5p is selected from the group consisting of miR-34a-5p, a miR-34a-5p variant, and agent inducing the expression of miR-34a-5p and an agent inducing the expression of a variant of miR-34a-5p,
- the agent inducing the expression of miR-375-3p is selected from the group consisting of miR-375-3p, a miR-375-3p variant, and agent inducing the expression of miR-375-3p and an agent inducing the expression of a variant of miR-375-3p,
- the agent inducing the expression of miR-509-5p is selected from the group consisting of miR-509-5p, a miR-509-5p variant, and agent inducing the expression of miR-509-5p and an agent inducing the expression of a variant of miR-509-5p,
- the agent inducing the expression of miR-879-5p is selected from the group consisting of miR-879-5p, a miR-879-5p variant, and agent inducing the expression of miR-879-5p and an agent inducing the expression of a variant of miR-879-5p,
- the agent inducing the expression of miR-466i-5p is selected from the group consisting of miR-466i-5p, a miR-466i-5p variant, and agent inducing the expression of miR-466i-5p and an agent inducing the expression of a variant of miR-466i-5p, and
- the agent inducing the expression of miR-434-5p is selected from the group consisting of miR-434-5p, a miR-434-5p variant, and agent inducing the expression of miR-434-5p and an agent inducing the expression of a variant of miR-434-5p.
